# EUROPEAN PATENT SPECIFICATION

(11) **EP 1 278 850 B1**
(45) Date of publication and mention of the grant of the patent: **13.06.2007**
(21) Application number: 01949287.5
(22) Date of filing: 14.04.2001
(51) Int. Cl.: C12N 15/12, C07K 14/705, C12Q 1/68, G01N 33/52, G01N 33/50

(54) **EDG8 RECEPTOR, ITS PREPARATION AND USE**
EDG8-REZEPTOR, DESSEN HERSTELLUNG UND VERWENDUNG
RECEPTEUR EDG8, SA PREPARATION ET SON UTILISATION

(30) Priority: 26.04.2000 EP 00108858; 01.08.2000 EP 00116589
(43) Date of publication of application: 29.01.2003
(73) Proprietor: Sanofi-Aventis Deutschland GmbH, 65929 Frankfurt am Main (DE)
(72) Inventor: KOSTENIS, Evi, 60594 Frankfurt am Main (DE); GASSENHUBER, Johann, 65185 Wiesbaden (DE)
(86) International application number: PCT/EP2001/004283
(87) International publication number: WO 2001/081573

(56) References cited:
- EP-A- 1 090 925
- WO-A-00/11166
- WO-A-00/22129
- WO-A-01/04139
- WO-A-99/19513
- DATABASE EMBL [Online] EBI; ID AC011461, AC AC011461, 8 October 1999 (1999-10-08) "Homo sapiens chromosome 19 clone CTC-429L19, WORKING DRAFT SEQUENCE, 4 ordered pieces" XP002176772
- GLICKMAN M ET AL.: "Molecular cloning, tissue-specific expression, and chromosomal localization of a novel nerve growth factor-regulated G-protein-coupled receptor, nrg-1." MOL. CELL. NEUROSCI., vol. 14, no. 2, August 1999 (1999-08), pages 141-152, XP000939383 cited in the application
- AN S ET AL.: "Signaling mechanisms and molecular characteristics of G-protein-coupled receptors for lysophosphatidic acid and sphingosine 1-phosphate" JOURNAL OF CELLULAR BIOCHEMISTRY - SUPPLEMENT, vol. 30/31, December 1998 (1998-12), pages 147-157, XP002127866
- HLA T ET AL.: "Sphingosine-1-phosphate signalling via the EDG-1 family of G-protein-coupled receptors" ANN N Y ACAD SCI, vol. 905, April 2000 (2000-04), pages 16-24, XP000939014
- DATABASE EM_HUM [Online] EMBL; ID AF317676, AC AF317676, 6 December 2000 (2000-12-06) IM D ET AL.: "Homo sapiens sphingosine 1-phosphate receptor Edg-8 gene, complete cds" XP002176773
- IM D-S ET AL.: "Characterization of a novel sphingosine 1-phosphate receptor, Edg-8" J. BIOL. CHEM., vol. 275, no. 19, 12 May 2000 (2000-05-12), pages 14281-14286, XP000939039

## Description

The present invention relates to a method for identifying a compound which binds to EDG8 polypeptide comprising an amino acid sequence according to SEQ ID NO: 2 or encoded by a polynucleotide sequence according to SEQ ID NO: 1 in an antagonistic manner with respect to sphingosine 1-phosphate (S1P), lysophosphatidic acid (LPA) and/or dihydrosphingosine 1-phosphate (DHS1P) comprising a] contacting a cell expressing said EDG8 polypeptide and cotransfected with Gα₁₆ or Gα_{qi5}, or a part of such a cell, with S1 P, and/or LPA and/or DHS1 P and a candidate compound, and b] assessing the ability of said compound to bind to said EDG8 polypeptide in an antagonistic manner with respect to S1P, LPA and/or DHS1P.

In an effort to identify new G-protein coupled receptors of the EDG (endothelial differentiation gene)-family a novel member of the EDG-family of G-protein coupled receptors, Human EDG8, was identified. The full-length clone was isolated and studies on chromosomal mapping, tissue expression and identification as a functional cellular receptor for sphingosine 1-phosphate were performed. Taken together, the data provide compelling evidence that EDG8 is the fifth receptor for sphingosine 1-phosphate, exclusively expressed in peripheral tissues, its presence in endothelial cells being responsible for the broad tissue distribution.

The lysolipid phosphate mediators lysophosphatidic acid (LPA) and sphingosin 1-phosphate (S1P) have attracted increasing attention as modulators of a variety of important biological functions (Moolenaar et al., 1997; Morris, 19.99; Lynch and Im, 1999) and their list of biological activities is continuously growing.
Among the biological responses to LPA is platelet aggregation (Jalink et al., 1994; Siess et al., 1999; Gueguen et al., 1999), smooth muscle contraction (Tokumura et al., 1980), in vivo vasoactive effects (Tokumura et al., 1995), chemotaxis (Jalink et al., 1993), expression of adhesion molecules (Lee et al., 1998b; Rizza et al., 1999), increased tight junction permeability of endothelial cells (Schulze et al., 1997), induction of stress fibers (Gohla et al., 1998) and many others (for review see Moolenaar et al., 1997). The biochemical signalling events that mediate the cellular effects of LPA include stimulation of phospholipases, mobilization of intracellular Ca²⁺, inhibition of adenylyl cyclase, activation of phosphatidylinositol 3-kinase, activation of the Ras-Raf-MAP kinase cascade and stimulation of Rho-GTPases (Moolenaar et al., 1997).
S1P, in particular, is implicated in cell proliferation, modulation of cell motility (reviewed in Hla et al., 1999) induction/suppression of apoptosis (Hisano et al., 1999; Xia et al., 1999), angiogenesis (Lee et al., 1999), tumor invasiveness (Sadahira et al., 1992), platelet activation (Gueguen et al., 1999) and neurite retraction (Postma et al., 1996). Cellular signalling by S1P involves activation of PLCβ and subsequent intracellular Ca²⁺ release (van Koppen et al., 1996; Meyer zu Heringdorf et al., 1997; Yatomi et al., 1997a; Noh et al., 1998; Ancellin and Hla, 1999), activation of MAP-kinases (Wu et al., 1995; Lee et al., 1996; An et al., 2000), activation of inward rectifying K⁺-channels (van Koppen et al., 1996; Bünemann et al., 1996) and inhibition and/or activation of adenylyl cyclase (Lee et al., 1996).
Both, LPA and S1P are recognized to signal cells through a set of G-protein coupled receptors (GPCRs) known as EDG (endothelial differentiation gene)-receptors. The EDG-family of GPCRs currently comprises seven human members (EDG1-7) that fall into two major groups depending on their preference for the activating lipid-ligand: EDG1, 3, 5 and 6 preferentially interact with S1 P (Yatomi et al., 1997b; Lee et al., 1998a,b; Ancellin and Hla, 1999; Yamazaki et al., 2000; Van Brocklyn et al., 2000), EDG2, 4 and 7 preferentially interact with LPA (An et al., 1998; lm et al., 2000).
The assignment of specific biological functions to certain receptor subtypes is hampered by the fact that EDG-receptors are expressed in an overlapping fashion (Rizza et al., 1999; Lee et al., 1999), they activate multiple and in part redundant signal transduction pathways (Lee et al., 1996; Ancellin and Hla, 1999; Kon et al., 1999; An et al., 2000), the selectivity for their activating ligands is not absolute (Lee et al., 1998b), and medicinal chemistry is only poorly developed in that specific antagonists for dissecting the pharmacology of the individual subtypes are not available yet.
An important step to shed more light on the biological role of the individual receptor subtypes would be to identify the complete set of receptors that respond to the phospholipid mediators S1P and LPA.

WO 00/11166 discloses a member of the superfamily of G-protein-coupled receptors named 14274 which is a further member of the EDG, receptor familly.

The present invention relates to a method for identifying a compound which binds to EDG8 polypeptide comprising an amino acid sequence according to SEQ ID NO: 2 or encoded by a polynucleotide sequence according to SEQ ID NO: 1 in an antagonistic manner with respect to sphingosine 1-phosphate (S1P), lysophosphatidic acid (LPA) and/or dihydrosphingosine 1-phosphate (DHS1P) comprising
a] contacting a cell expressing said EDG8 polypeptide and cotransfected with Gα₁₆ or Gα_{qi5}, or a part of such a cell, with S1P, and/or LPA and/or DHS1P and a candidate compound, and
b] assessing the ability of said compound to bind to said EDG8 polypeptide in an antagonistic manner with respect to S1P, LPA and/or DHS1P.

Preferably, the method for identifying compounds further includes determining whether the candidate compound effects a signal generated by activation of said EDG8 polypeptide in an antagonistic manner at the surface of the cell, wherein a candidate which effects production of said signal is identified as an antagonist. The polynucleotide sequence may be part of an expression vector.

Preferably, the polynucleotide is DNA or RNA.

In another aspect, the specification relates to a process for producing the EDG8 polypeptide wherein a host cell comprising the expression system is cultured under conditions sufficient for the production of said polypeptide.

Preferably, the said polypeptide is expressed at the surface of said cell.

The specification relates also to cells produced by this process.

The process preferably further includes recovering the polypeptide from the culture.

In another aspect, the specification relates to a process for producing a cell which produces the EDG8 polypeptide comprising transforming or transfecting a host cell with the expression system such that the host cell, under appropriate culture conditions, produces the EDG8 polypeptide.

One characteristic functionality of human EDG8 is that the polypeptid is a S1P receptor; it responds to S1P and optionally to related phospholipids like DMS1P or LPA.

In another embodiment of the invention, the method for identifying compounds further includes determining whether the candidate compound effects a signal generated by activation of the EDG8 polypeptide at the surface of the cell, wherein a candidate compound which effects production of said signal is identified as an antagonist.

The specification in addition, relates to a method of preparing a pharmaceutical composition comprising
a) identifying a compound which is an agonist or an antagonist of EDG8,
b) preparing the compound, and
c) optionally mixing the compound with suitable additives.

The specification relates to a pharmaceutical, comprising as active ingredient for example such identified compound, an EDG8 polypeptid or a polynucleotide encoding for EDG8.

In particular, the specification relates to a pharmaceutical, that can be used for the prevention and/or treatment of diseases associated with EDG8/S1P signal transduction, for example diseases associated with endothelial dysfunction such as for example Atheriosclerosis, Shoke, Hypertonie, coronary syndroms, cancer, thrombolylic diseases, affected wound healing and diseases accompanied by increased cell death. In another aspect of the specification, such pharmaceutical can be used for the prevention and/or treatment of diseases associated with a dysregulation of angiogenesis, such as for example tumor growth, rheumatical arthritis and diabetic setinopathy.

The study, reported about the cloning, chromosomal mapping, tissue expression and functional identification as a receptor for S1 P of a novel GPCR, EDG8, the fifth functional receptor for sphingosine 1-phosphate.

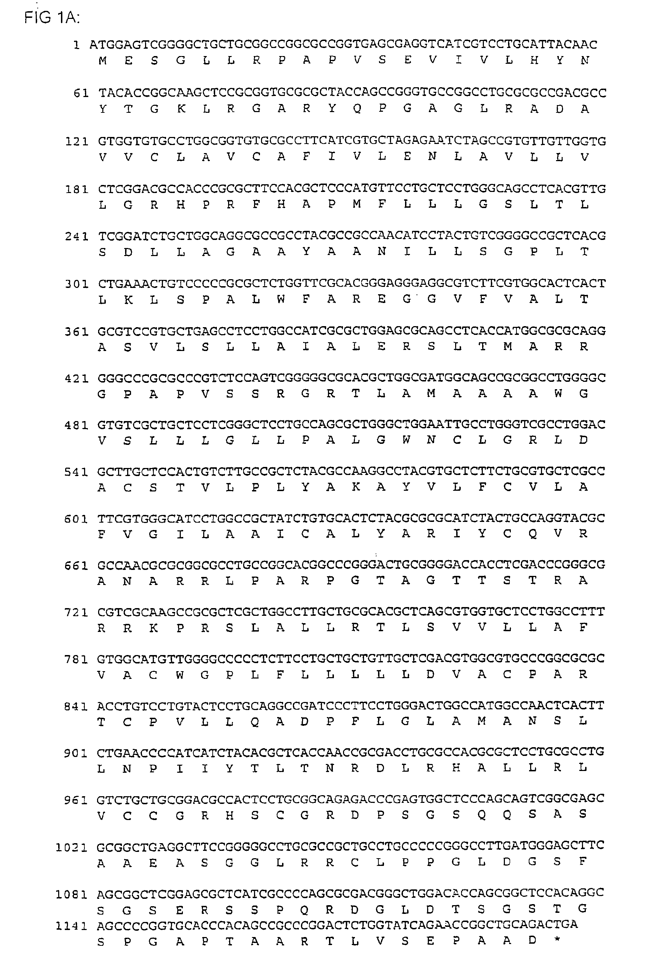

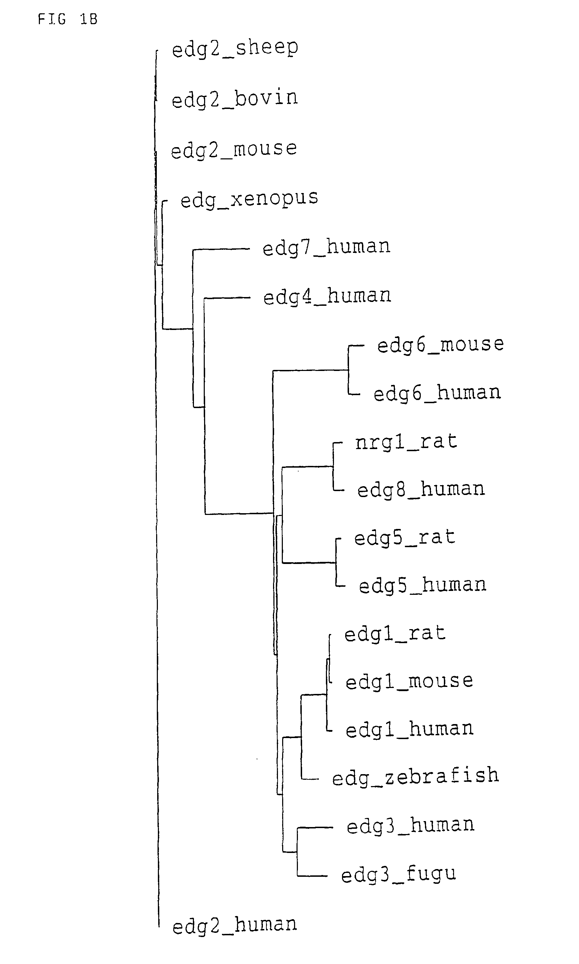

In an effort to identify new G-protein coupled receptors of the EDG-family a database search with alignments of the currently known 18 members of this receptor family was performed, comprising human EDG1-7 sequences up to the putative EDGs from Xenopus and Zebra-fish. A multiple alignment of these sequences was created by CLUSTALW and used in a PSI-BLAST search to scan translated versions of human genomic DNA sequences, which were publicly available in the different EMBL sections. For translation of DNA into protein sequences, individual protein files within two respective STOP-codon were created and all proteins shorter than 50 amino acids were ignored. As the majority of GPCRs is unspliced searching for GPCRs within genomic sequences should bring about novel receptor proteins.
Performing a PSI-BLAST search, the various cDNAs and genomic contigs, respectively, for the human EDG1-7 receptors were identified, and an additional genomic hit, highly homologous to human EDG5 (51 % homology), termed EDG8. The nucleotide and amino acid sequence of the new putative GPCR are depicted in Fig.1A.
Hydropathy analysis (hydrophobicity plot not shown) suggests a seven transmembrane protein with three alternating extra- and intracellular loops, assumed to be the heptahelix structure common to GPCRs.
To shed more light on the relationships involved in the molecular evolution of the EDG-receptor family, a grow tree phylogram was constructed using the neighbor joining method (GCG software) (Fig.1B) (Comparison of amino acid sequences). According to this phylogenetic tree, the human EDG-family can be divided into two distinct groups: EDG1, 3, 5 and 6 belonging to one, EDG2, 4 and 7 belonging to the other group. These two groups are discriminated further by their preference for different lipid ligands: EDG1, 3, 5, 6 are preferentially stimulated by sphingosin 1-phosphate (S1P) (Yatomi et al., 1997b; Lee et al., 1998a,b; Ancellin and Hla, 1999; Yamazaki et a(., 2000; Van Brocklyn et al., 2000), EDG2, 4 and 7 by lysophosphatidic acid (LPA) (Hecht et al., 1996; An et al., 1998; Im et al., 2000). The newly identified EDG8 exhibited highest similarity (86.8% aminoacid identity) to the rat nrg1-protein (Fig. 1B), a GPCR recently cloned by EST-expression profiling from a rat PC12 cell library (Glickman et al., 1999), which probably represents the rat homologue of human EDG8. In the report of Glickman, however, the authors did not address the question of the activating ligand of this receptor. The high similarity between EDG8 and the known sphingosin 1-phosphate (S1P) receptors EDG1, 3 and 5 (48-51%) (Fig. 1C) led to test the hypothesis that EDG8 may be a functional S1P-receptor.
In testing for S1 P receptor activity, the EDG8 cDNA was introduced into chinese hamster ovary (CHO) cells by transient transfection. CHO cells were chosen as they exhibit minimal responses to sphingosin 1-phosphate in concentrations up to 1 µM but respond to S1P after transfection with the S1P preferring receptors EDG 1, 3 and 5 (Okamoto et al., 1998; Kon et al., 1999). To test functional receptor activity the mobilization of [Ca²⁺]ᵢ was monitored for three reasons:
1.) S1 P has been reported to increase Ca²⁺ in many cell types (Ghosh et al., 1990; Zang et al., 1991; Durieux et al., 1993; Chao et al., 1994; Gosh et al., 1994; Mattie et al., 1994; Meyer zu Heringdorf et al., 1996; Okajima et al., 1996; van Koppen et al., 1996; Törnquist et al., 1997; Yatomi et al., 1997; Noh et al., 1998; An et al., 1999)
2.) the identification of EDG1, 3, 5 and 6 as receptors for S1P has provided the molecular basis for a GPCR mediated mechanism and the receptors are known to mediate intracellular Ca²⁺-release through either PTX-sensitive Gαᵢ proteins or the PTX-insensitive Gα_{q/11} pathway (Okamoto et al., 1998; Kon et al., 1999; Gonda et al., 1999)
3.) all currently known S1P-responding EDG-receptors (except EDG6) are present in endothelial cells (A. Niedernberg et al., submitted), in which intracellular Ca²⁺ release is a major pathway in the generation of NO, an important factor in vascular biology. Thus, identification of the complete set of S1P receptors, involved in intracellular Ca²⁺ mobilization could help clarify the role of the individual subtypes in endothelial cell signalling.

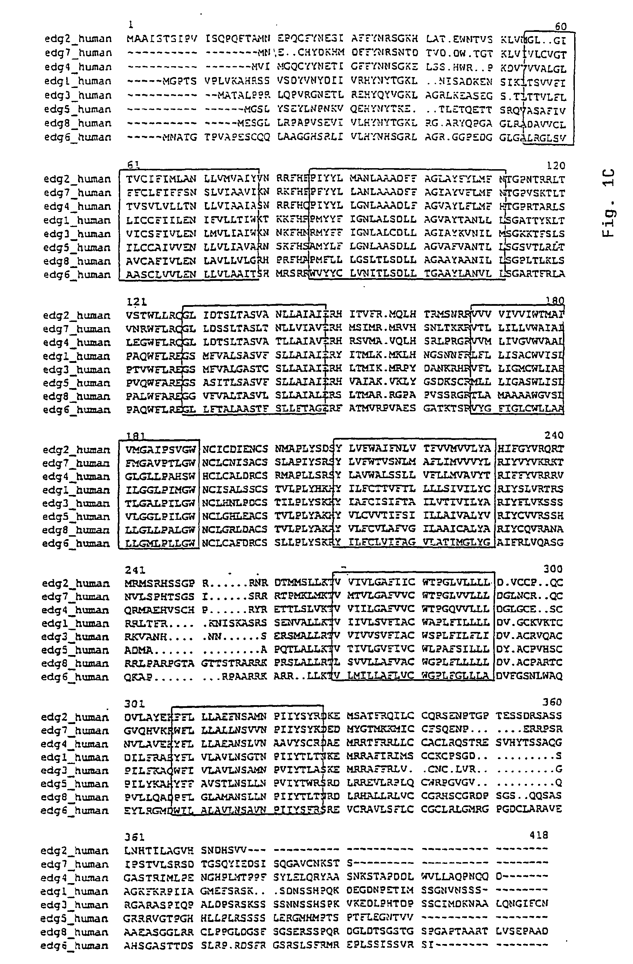

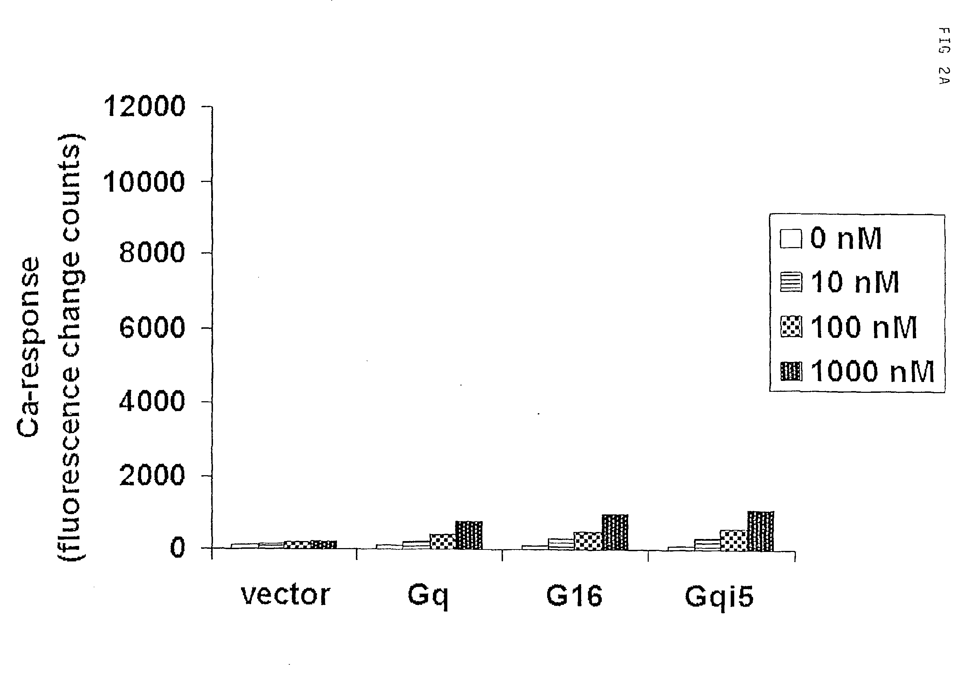

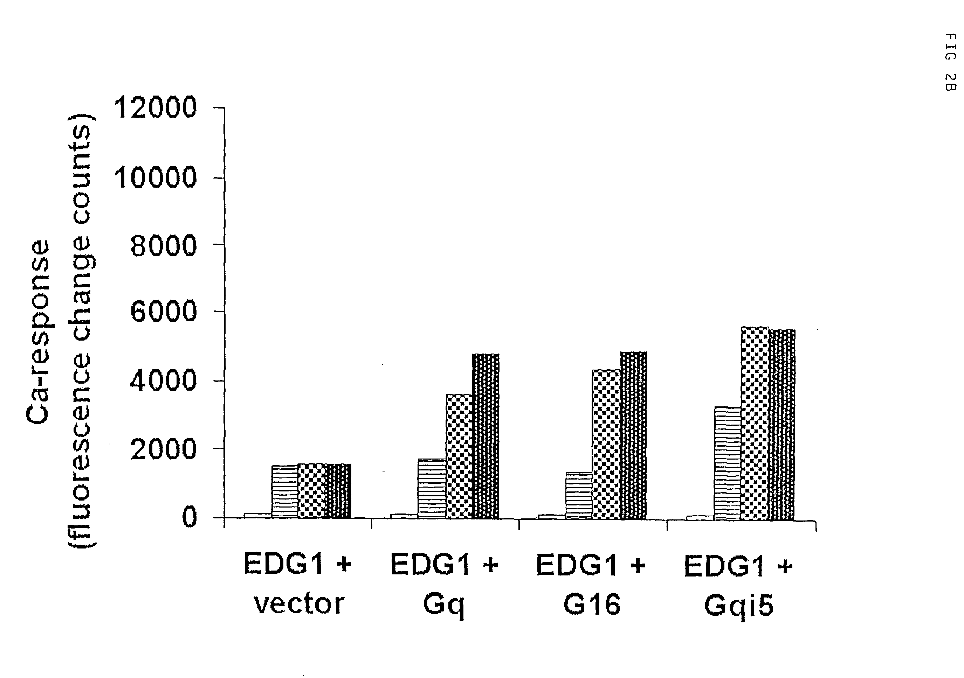

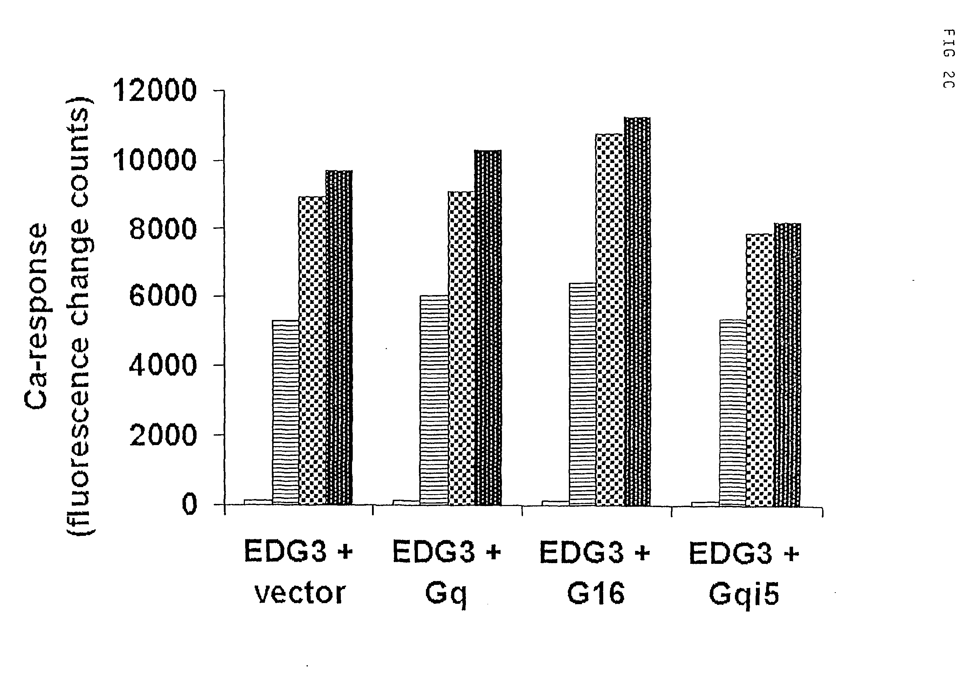

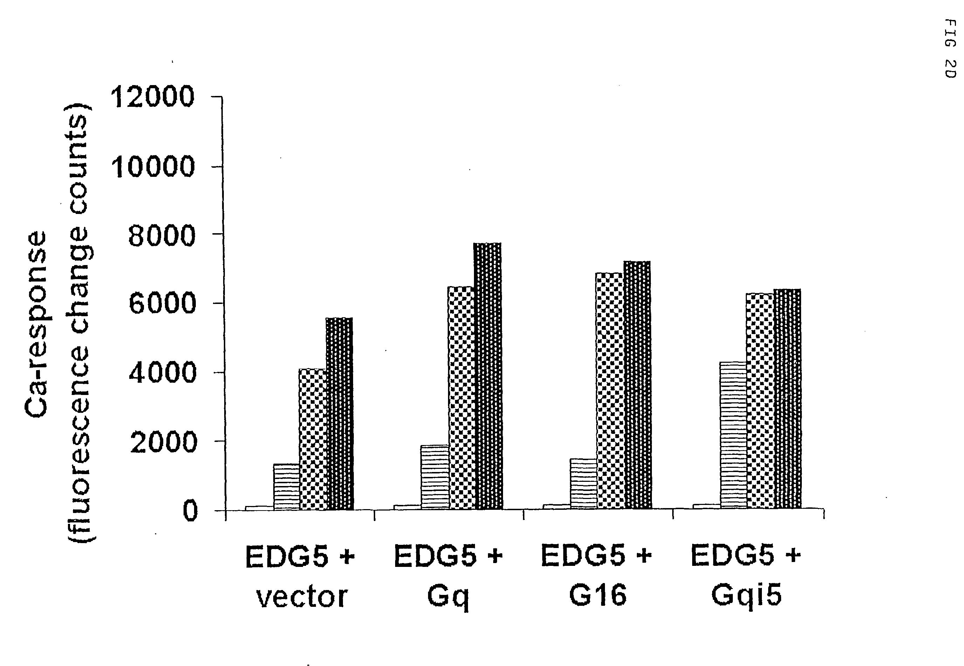

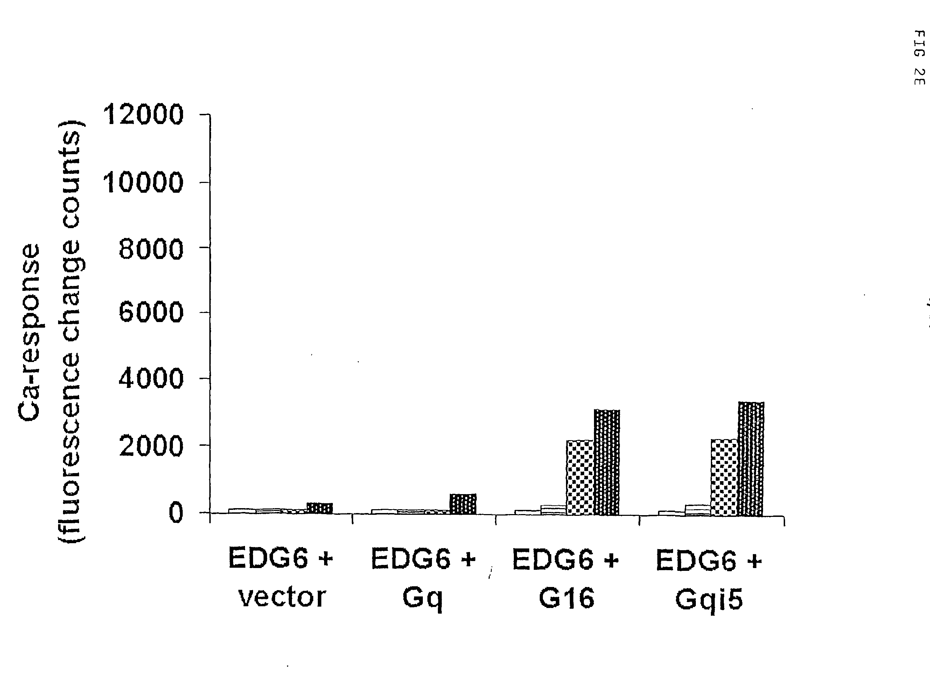

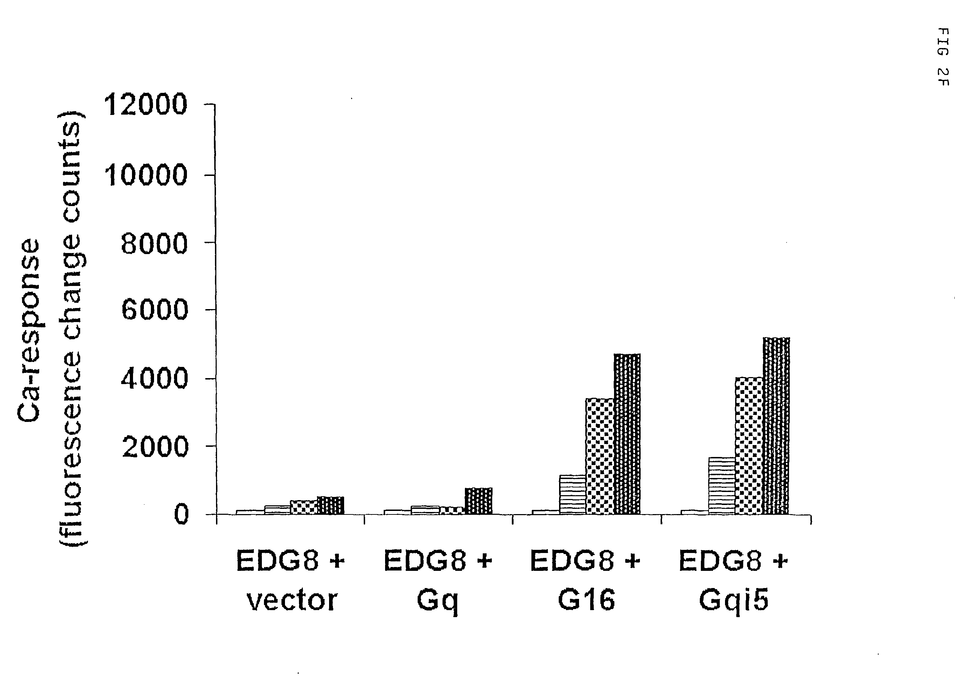

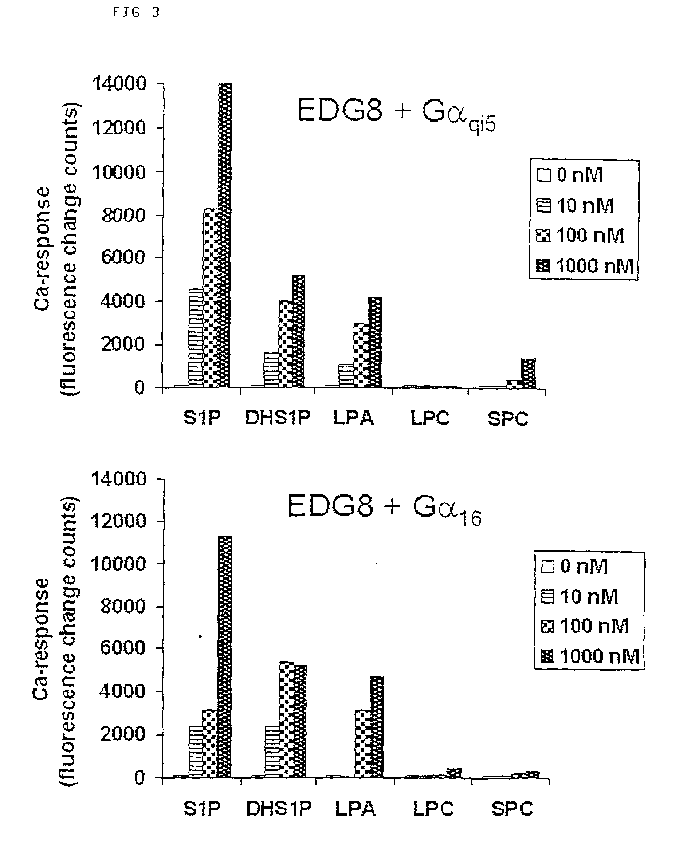

Fig.2 depicts measurement of the intracellular Ca²⁺ concentration, mediated by S1P via the putative S1P receptor EDG8. For sake of comparison, the S1P-receptors EDG1, 3, 5, and 6, which have been reported to mobilize [Ca²⁺]ⱼ, were included. [Ca²⁺]ᵢ were recorded as real time measurements using the Fluorescence plate imaging reader (FLIPR, Molecular Devices). Initially, CHO cells transfected with empty vector DNA were stimulated with different concentrations of S1P (10, 100,1000 nM). None of the applied S1P concentrations was capable of eliciting significant rises in intracellular Ca²⁺ (Fig. 2A), suggesting that S1P receptors are not expressed in CHO cells or, if expressed, are unable to signal via the endogeneous Gα_{q} pathway. To address this issue, the G protein chimera Gα_{qi5}, which confers onto Gi coupled receptors the ability to stimulate the Gq pathway, and Gα₁₆, which links Gi- and Gs coupled receptors to PLCβ and subsequent intracellular Ca²⁺-mobilization were used. Upon stimulation with S1P, G_{qi5}- and G₁₆- transfected CHO cells did not give rise to significant increases in [Ca²⁺]i (Fig. 2A). However, transient transfection of CHO-cells with the cDNAs coding for the EDG1, 3 and 5 receptor conferred S1P-responsiveness to the cells: it was confirmed that EDG1, 3 and 5 mobilize [Ca²⁺]i in response to S1 P (Fig. 2B, C, D) (Kon et al., 1999). As already known for a large number of Gq-coupled receptors, coexpression of Gα_{q} augments the EDG1 and 5-mediated Ca²⁺-response as compared with the Ca²⁺ signal induced by stimulation of endogeneous Gα_{q}. In case of EDG3, additional exogeneously added Gα_{q} did not further improve the signal intensity. These results are in agreement with the findings reported by Kon et al. (1999), who showed that the EDG3-subtype causes the most robust enhancement of intracellular Ca²⁺.
In case of EDG6, Yamazaki et al. (2000) obtained an S1P-induced mobilization of [Ca²⁺]ᵢ but we failed to detect a significant Ca²⁺ increase above basal levels in the absence of any cotransfected G-protein α subunit (Fig. 2E). The reason for this discrepancy could be the cellular background (CHO cells in this study vs. K562 cells in Yamazaki et al.), as they reported a pertussis toxin (PTX)-sensitive Ca²⁺-response, indicating the involvement of Gi-type G-proteins. In this case the Ca²⁺ signal would be elicited by βγ, released from activated Gαᵢßγ heterotrimers. The Gαᵢ-induced Ca²⁺ signals are known to be much smaller in intensity as compared with the Ca²⁺ signals induced by bona-fide Gq-linked receptors (Kostenis et al., 1997). It may be that detection of such [Ca²⁺]i concentrations is beyond the sensitivity of the FLIPR system. EDG8 did not release [Ca²⁺]ᵢ when stimulated with S1P (10, 100, and 1000 nM) (Fig.2F), but gained the ability to mobilize Ca²⁺ upon cotransfection with Gα₁₆, a G-protein α subunit, known to couple GPCRs from different functional classes to the Gq-PLCβ pathway or Gα_{qi5}, a mutant G-protein α subunit that confers onto Gi-linked receptors the ability to stimulate Gq (Conklin et al., 1993). These results show that EDG8 is a functional receptor for S1P and that EDG8-induced Ca²⁺ responses are due to a non-Gq pathway, probably the activation of phospholipase Cβ2 by βγ subunits of the Gi proteins. Furthermore, these results provide additional evidence that the S1P-preferring EDG-receptors couple differentially to the Gq and Gi pathways: EDG3 ist the most potent Ca²⁺-mobilizing receptor and overexpression of Gα_{q} does not further improve Ca²⁺ signalling; EDG1 and 5 induce moderate Ca²⁺-increases, that can be significantly improved by cotransfection of Gα_{q} or a chimeric Gα_{qi5} protein; EDG8-mediated Ca²⁺-responses require cotransfection of Gα_{qi5} or Gα₁₆. To check, whether the EDG8 receptor also reacts to related lysophospholipid mediators, we examined the abilities of lysophosphatidic acid (LPA), dihydrosphingosin 1-phosphate (DHS1P), sphingosylphosphorylcholine (SPC) and lysophosphatidylcholine (LPC) to increase intracellular Ca²⁺ in CHO cells transiently transfected with the EDG8 receptor and the G-protein α subunits Gα₁₆ and Gα_{qi5} (Fig.3). Besides S1P, which was the most potent activator of EDG8, LPA and DHS1P evoked [Ca²⁺]ᵢ increases in concentrations of 100 and 1000 nM. SPC and LPC, respectively, failed to generate any significant response in concentrations up to 1 µM. These data show that EDG8 is a S1 P preferring receptor, but also responds to related phospholipids like DHS1P or LPA, as has also been reported for EDG1, which is a high affinity receptor for S1P and a low affinity receptor for LPA (Lee et al., 1998b). Therefore, EDG8 receptor has the characteristic functionality to respond to S1 P and related phospholipids like DMS 1P or LPA. The response to S1P and other related phospholipides can for example be determined as described in Example 3. Cells containing the respective Gα can be obtained as described in Example 2.

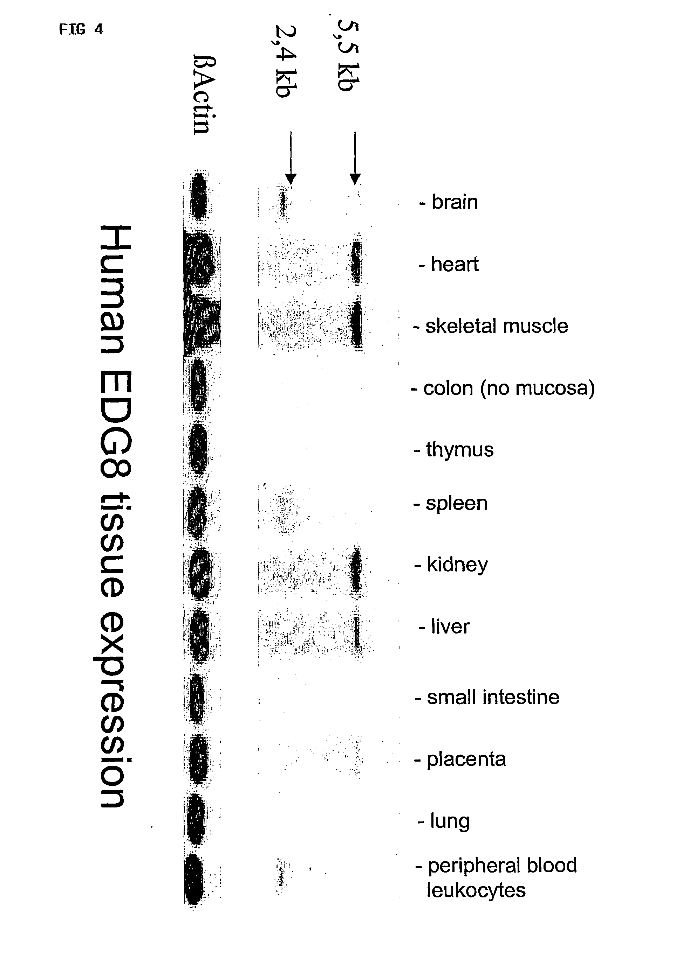

Next, the expression pattern of the EDG8 gene in human tissues was investigated by Northern blot analysis (Fig.4). Tissues positive for EDG8 RNA were skeletal muscle, heart and kidney, lower abundance of RNA was seen in liver and placenta, no signal was detected in brain, thymus, spleen, lung and peripheral blood leukocytes. In all tissues a single RNA transcript of 5.5 kb was observed after hybridization with a DIG-labelled EDG8 antisense RNA probe. EDG8 exhibits highest similarity to the rat nrg1-GPCR (Glickman et al., 1999) with an amino acid identity of 86.8% (Fig.1B) suggesting that it may be the human homolog of the rat nrg1 protein. However, the expression pattern of human EDG8 is quite different from the rat nrg1-receptor,

which is found almost exclusively in brain (Glickman et al., 1999). This finding suggests that EDG8 may represent a closely related but entirely different receptor from nrg1, rather than the human homolog. Never the less, it does not rule out the possibility that EDG8 and nrg1 are homologs with entirely different, species-dependent expression patterns.

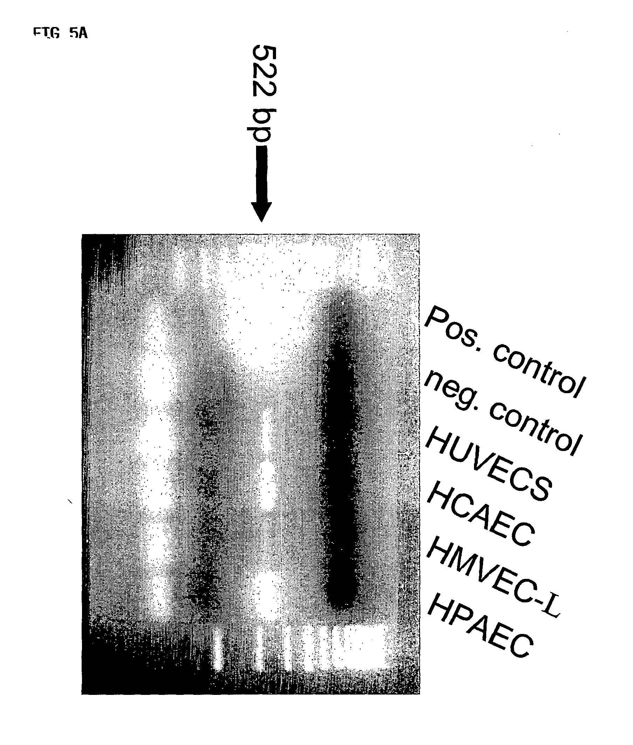

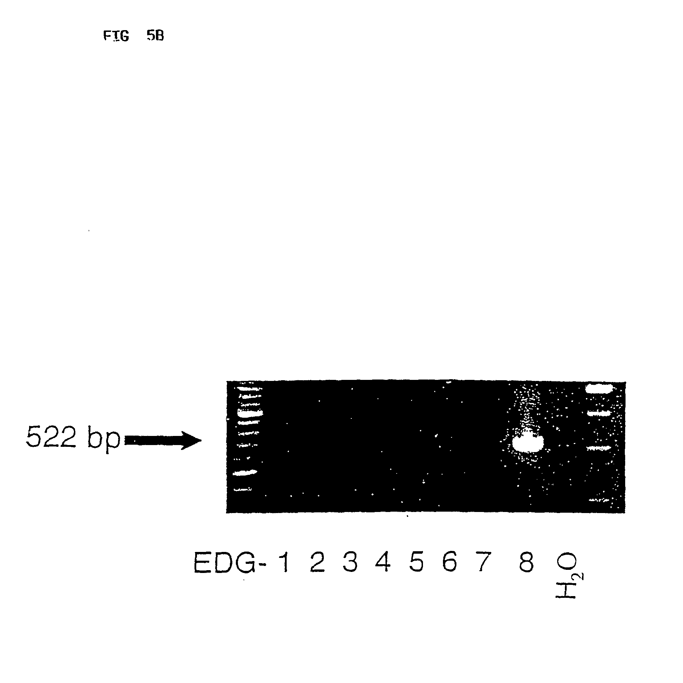

As the first member of the EDG-family of GPCRs - EDG1 - was originally cloned as an endothelial differentiation gene from phorbol-myristic-acetate-treated differentiating human endothelial cells (HIa and Maciag, 1990) and subsequently cloned from a human umbilical vein endothelial cell library exposed to fluid shear stress as an upregulated gene it is reasonable to assume that EDG receptors play an important role in the regulation of endothelial function. Therefore, the presence of EDG8 transcripts in several human endothelial cell lines was analyzed. RT-PCR analysis of human umbilival vein endothelial cells (HUVECs), human coronary artery endothelial cells (HCAECs), human microvascular endothelial cells of the lung (HMVEC-L) and human pulmonary artery endothelial cells (HPAEC) revealed EDG8 expression in all cell lines tested (Fig.5A). In Fig.5B it is shown that EDG8 specific primers indeed solely amplify EDG8 sequences and none of the related EDG1-7 sequences. These findings suggest that the presence of EDG8 in different peripheral organs may be due to its localization in endothelial cells; it does not rule out, however, that EDG8 transcripts occur in cell types other than endothelial cells.
The expression of EDG8 in addition to EDG1, 3, and 5 (Rizza et al., 1999) in HUVECS and several other endothelial cell lines is intriguing in view of all the reports regarding S1P effects on endothelial cell signalling. Hisano et al. (1999) reported that S1P protects HUVECS from apoptosis induced by withdrawal of growth factors and stimulates HUVEC DNA synthesis; the authors derived a model for cell-cell interactions between endothelial cells and platelets but the S1P-receptor responsible for HUVEC-protection of apoptosis could not be identified. Rizza et al., 1999 reported that S1P plays a role in endothelial cell leukocyte interaction in that S1P induces expression of cell adhesion molecules in human aortic endothelial cells, allowing monocytes and neutrophils to attach. These effects were blocked by pertussis toxin, suggesting the involvement of a Gi-coupled S1P receptor. The responsible S1Preceptor subtype, however, could not be identified and the EDG8 receptor was not included at the time of this study. Expression profiling of all EDG receptors in individual cell lines and the use of EDG receptor subtype selective compounds will clearly be necessary to help determine the role of the individual S1P receptors in endothelial cell signalling mechanisms.

Finally, the mapping of EDG receptors in genomic sequences allowed to derive the chromosomal localization for four genes of this family (Tab.1). Interestingly, so far, four EDG-receptors including EDG8 are located on chromosome 19. In addition, the genomic sequence allowed the determination of the structure of the genes: the S1P-preferring receptors EDG1, 3, 5 and 8 are intronless as opposed to the LPA-preferring subtypes 2, 4 and 7, that contain an intron in the open reading frame in TMVI. These data suggest that in addition to the activating ligand and the degree of homology, the two subclasses of lysophospholipid receptors can be discriminated further by their genomic structure. The genomic structure of new potential EDG/LPA-receptor family members may also help predict the nature of the activating lipid ligand.

In conclusion, a new member of the EDG-family of G-protein coupled receptor, human EDG8, was isolated. This receptor functions as a cellular receptor for sphingosine 1-phosphate. EDG8 could exclusively be detected in peripheral tissues like skeletal muscle, heart and kidney and several human endothelial cell lines. It is conceivable that the expression in endothelial cells may account for the broad tissue distribution of this receptor. The existence of at least eight EDG-receptors for lysophospholipids suggests that receptor subtype selective agonists and antagonists will essentially be necessary for a better understanding of the biology of lysophospholipids and their respective receptors.

### Figure legends

Fig.1A: The nucleotide and deduced amino acid sequence of human EDG8. The deduced amino acid sequence is shown below the nucleotide sequence with the nucleotide positions indicated on the left.

Fig. 1B: Phylogenetic tree of the EDG-family of receptors. The phylogenetic tree depicted was derived by the neighbor joining method method performed with the GCG program.

Fig.1C: Alignment of the amino acid sequence of human EDG8 with the other EDG-family members. The amino acid sequence of EDG8 is compared with the EDG1-7 polypeptides (EDG1: accession number M 31210, EDG2: accession number U 80811, EDG3: accession number X 83864, EDG4: accession number AF 011466, EDG5: accession number AF 034780, EDG6: AJ 000479, EDG7: accession number AF 127138). The approximate boundaries of the seven putative transmembrane domains are boxed. Gaps are introduced to optimize the alignment.

Fig.2A-F: Mobilization of intracellular Ca²⁺ by S1P (10, 100 and 1000 nM) mediated by the EDG1,3,5,6 and 8 receptor in CHO cells, cotransfected with empty vector DNA as a control or the indicated G-protein α subunits.
A: S1P-induced Ca²⁺-response in CHO cells transfected with vector DNA alone or the G protein α subunits Gq, G16 and Gqi5. B-F: S1P-induced Ca²⁺-response in CHO cells transfected with the indicated EDG-receptor subtypes. Agonist-mediated changes of intracellular Ca²⁺ were measured with the FLIPR using the Ca²⁺-sensitive dye FLUO4 as described in Experimental procedures. Fluorescence of transfected cells loaded with FLUO4 was recorded before and after addition of S1P, applied in the indicated concentrations. Data are expressed as means of quadruplicate determinations in a single experiment. An additional experiment gave similar results.

Fig.3: Effects of S1 P, LPA and related lysophospholipid mediators on EDG8-mediated increase in intracellular Ca²⁺. CHO-cells were cotransfected with EDG8 and the G protein α subunits Gqi5 (upper panel) and G16 (lower panel) and rises in [Ca²⁺]ᵢ were recorded with the FLIPR as described in Experimental procedures. The different lipids were applied in concentrations of 10, 100 and 1000 nM, respectively. Data are means of quadruplicate determinations of a representative experiment. Two additional experiments gave similar results.

Fig.4: Northern blot analysis of EDG8 in human tissues. Poly(A)+ RNA (1µg) from various human tissues (human multiple tissue Northern blots, CLONTECH) was hybridized with probes specific to human EDG8 (upper panel) and β-actin (lower panel) on a nylon membrane. The origin of each RNA is indicated at the top, the molecular mass of standard markers in kilobases (kb) is shown on the left.

Fig.5A: Reverse transcriptase-polymerase chain reaction (RT-PCR) analysis of EDG8 in different human endothelial cell lines (HUVECS: human umbilical vein endothelial cells; HCAEC: human coronary artery endothelial cells; HMVEC-L: human microvascular endothelial cells from lung; HPAEC: human pulmonary artery endothelial cells). EDG8-specific transcripts were detected in all endothelial cell lines. Agarose gel electrophoresis of the PCR products after 35 cycles of amplification with the GC-melt kit (as described in Experimental Procedures) is shown. Amplification with EDG8-specific primers yields a 522 bp EDG8-fragment as indicated by the arrow. The EDG8 plasmid served as a template for the positive control, H₂O was used instead of plasmid DNA as a negative control.

Fig.5B: PCR analysis of EDG8 primers for specificity of amplification of EDG8 sequences. Primers, specific for the EDG8 sequence, were checked for potential amplification of the related EDG1-7 sequences, using the respective plasmids as templates. Agarose gel electrophoresis of the PCR products after 35 cycles of amplification with the GC-melt kit (as described in Experimental Procedures) is shown. The EDG8 specific 522 bp band occurred only when EDG8 was used as a template. H₂O was used instead of plasmid DNA as a negative control.

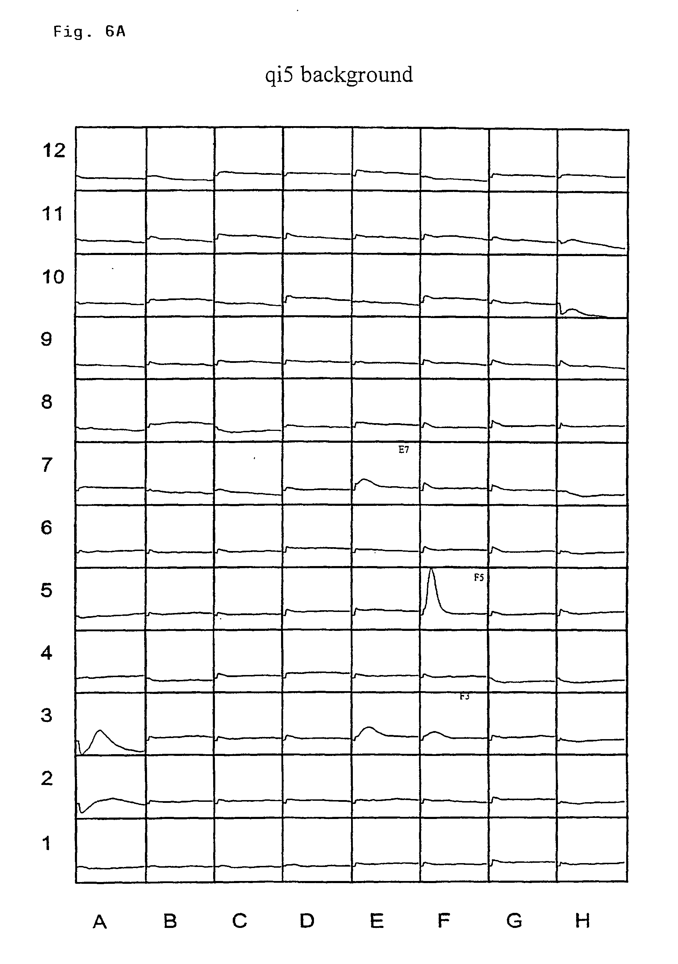

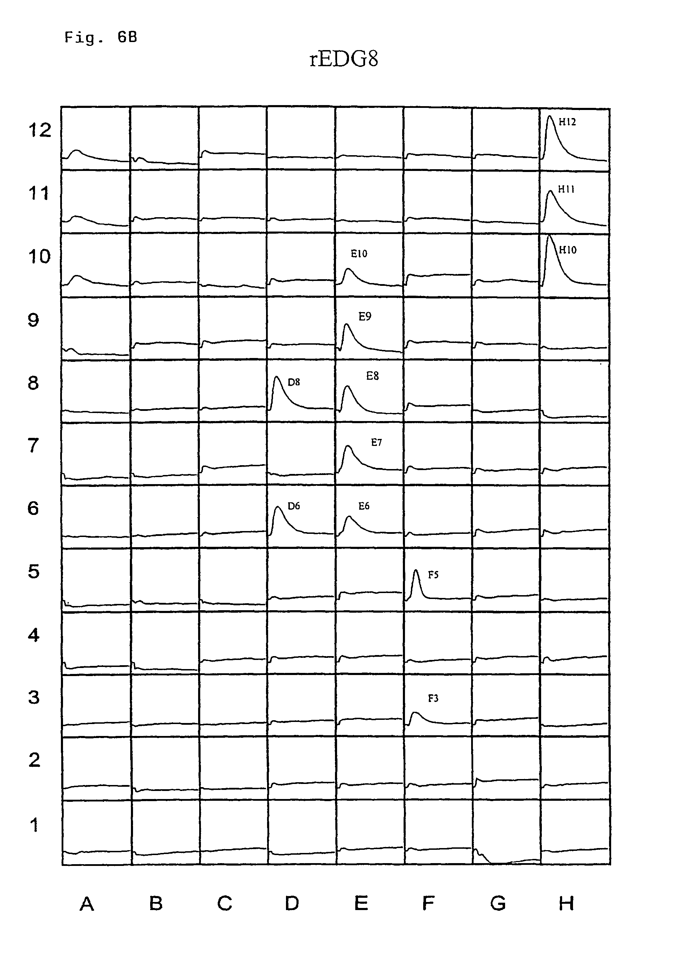

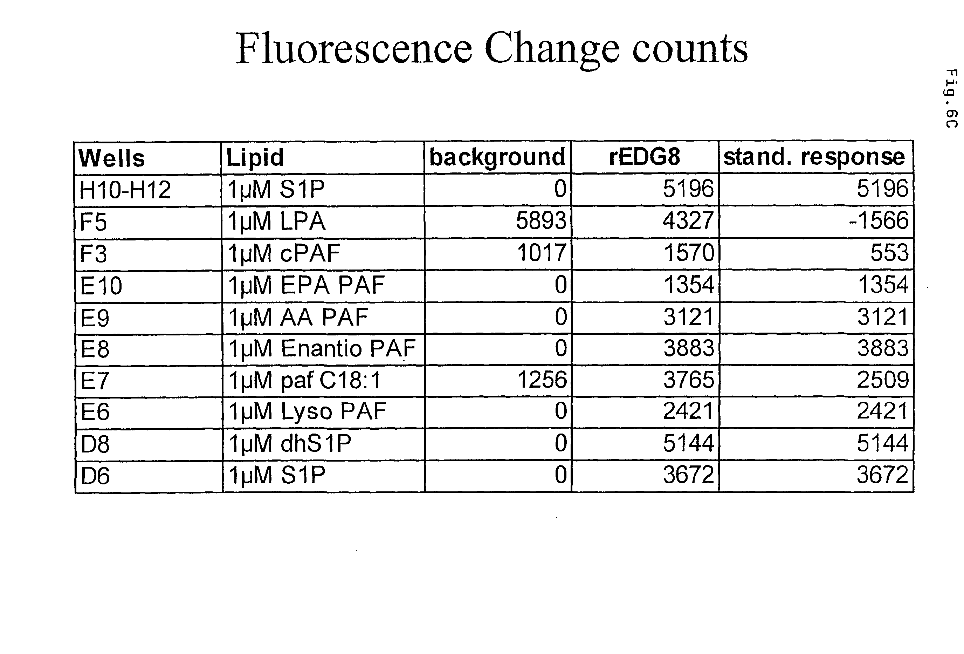

Fig.6: Experiments were performed according to example 3. Instead of lipids, a lipid library was used.

Fig.6A+B: Library plattes with rat EDG8 (r EDG8) and qi5.

Fig.6A: qi5 background.

Fig.6B: Measurement with rEDG8.

Fig.6C: Fluorescence change counts.

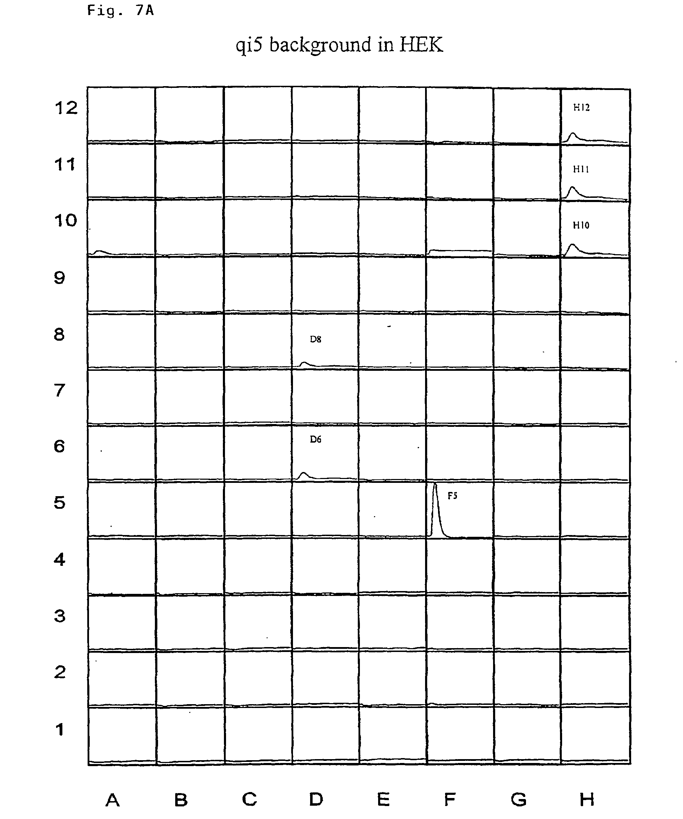

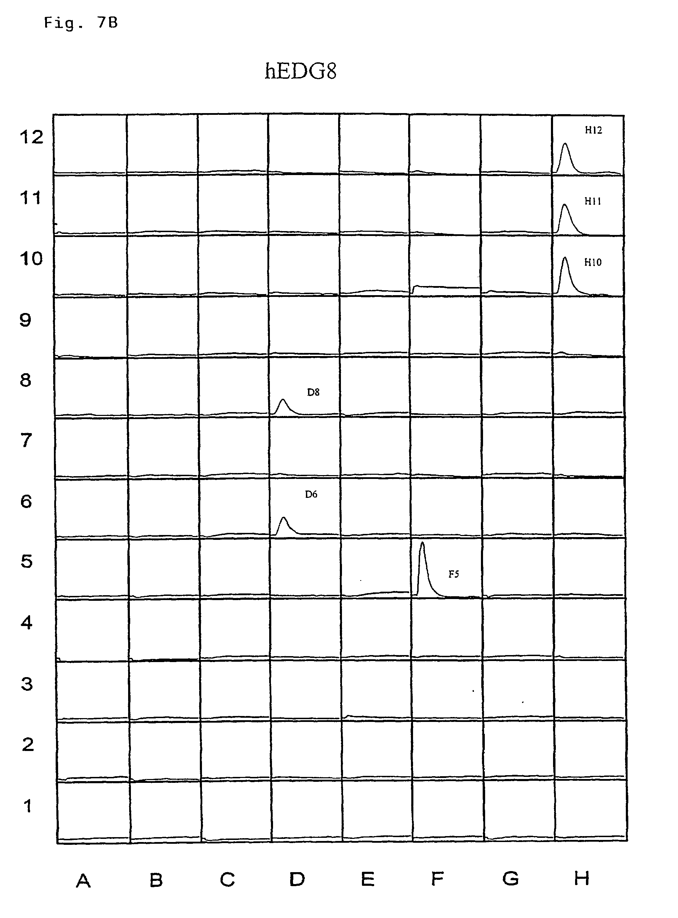

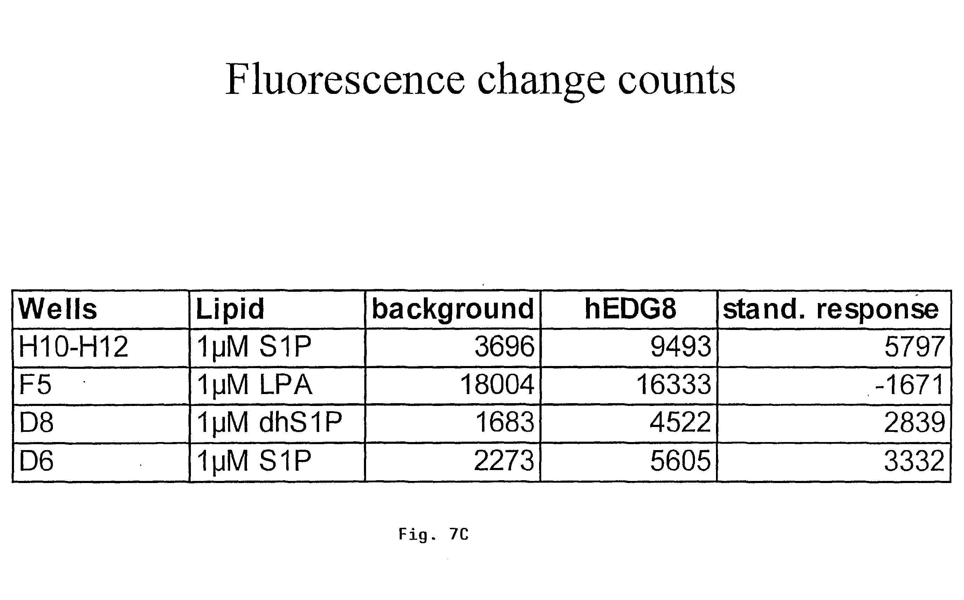

Fig.7: Experiments were performed according to example 3. Instead of Lipids, a lipid library was used.

Fig.7A+B: Library plates with human EDG8 (hEDG8) and qi5.

Fig.7A: q15 background.

Fig.7B: Measurement with hEDG8

Fig.7C: Fluourescence change counts.

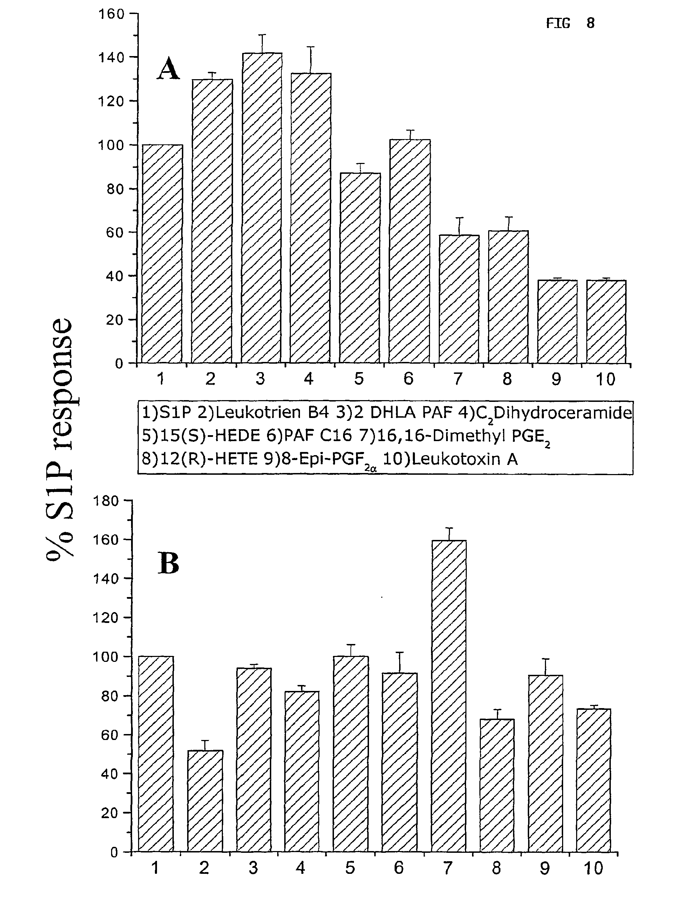

Fig.8: Antagonism of S1P activation of rat and human EDG8.
Transiently transfected CHO cells expressing rat EDG8 and Gα_{qi5} (A) and HEK 293 cells expressing human EDG8 and Gα_{qi5} (B) were incubated with test compounds, namely, 0.1 µM Leukotriene B4, 1 µM 2-DHLA-PAF (1-O-Hexadecyl-2-O-dihomo-γ-linolenoyl-sn-glycero-3-phophorylcholine), 1µM C₂ Dihydroceramide, 0.1 µM 15(S) HEDE (15(S)-Hydroxyeicosa-11Z,13E-dienoic acid), 1µM PAF C16 (1-0-Hexadecyl-2-O-acetyl-sn-glycero-3-phosphorylcholine), 1µM 16,16 Dimethyl PGE₂ (16,16-Dimethyl-Prostaglandin E₂) 12, 0.1 µM (R)-HETE (12(R)-Hydroxyeicosa-5Z,8Z,10E,14Z-tetraenioc acid), 1µM 8-epi-PGF_{2α} (8epi-Prostaglandin F_{2α}) 0.1 µM Leukotoxin A ((±) 9,10-EODE) or with solvent buffer for 3 min and then challenged with 1 µM S1P (sphingosine 1-phosphate). Peak fluorescence counts of cells preincubated with solvent buffer and then stimulated with 1 µM S1P were set 100 %. Fluorescence change counts were recorded with the FLIPR as described in detail in Experimetal procedures. Data are means ± SE of 2-3 independent experiments.

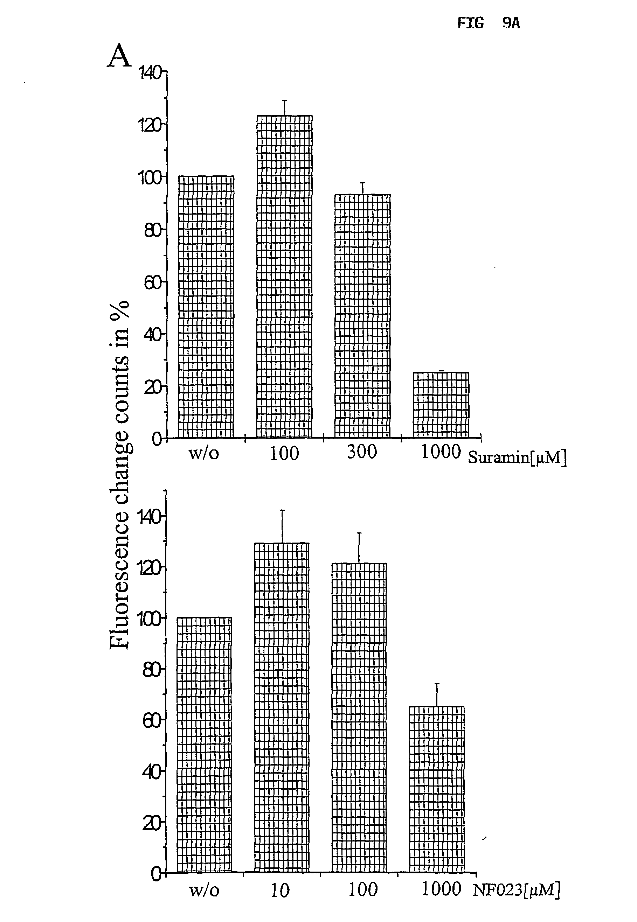

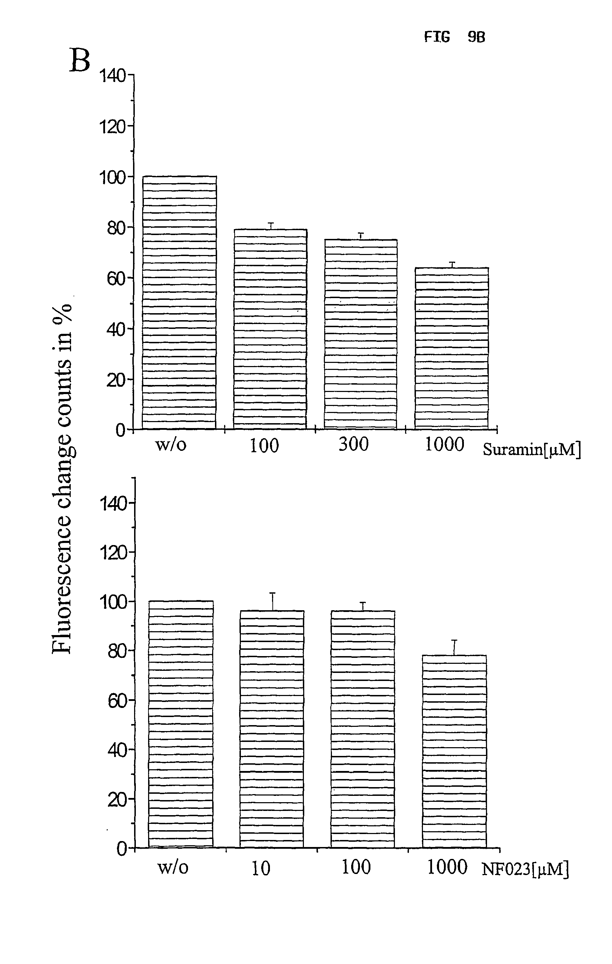

Fig.9: Inhibition of S1P mediated intracellular calcium release by suramin and NF023 (8,8'-(carbonylbis(imino-3,1-phenylene))bis-(1,3,5-naphatlenetrisulfonic acid)) in cells transiently cotransfected with with human EDG8 and Gα_{qi5} (A) and rat EDG8 and Gα_{qi5} (B). Transfected cells were first treated with the indicated concentrations of the inhibitor or solvent buffer for 3 minutes (NF023 and suramin did not show any effect on [Ca²⁺]ᵢ mobilization during the preincubation period). Cells were then stimulated with 1µM S1P and in [Ca²⁺]ᵢ measured with the FLIPR as described in the method section. Peak fluorescence counts were normalized and background responses of Gα_{qi5} - transfected cells were subtracted. S1P-mediated calcium release in the absence of inhibitor was set 100%. Data are means ± SE of 4-7 independent experiments.

**TABLE 1: Chromosomal localization, gene structure and accession number of the respective EDG genomic clones**

| Mapping of EDG receptors in genomic sequences allowed to derive a chromosomal assignment for EDG1, 2, 4-8. The chromosomal localization of EDG3 was obtained from Yamagutchi et al. (1996). Genomic sequences also revealed EDG1, 3, 5, 6 and 8 to be unspliced as opposed to EDG2, 4 and 7, which contain an intron in their open reading frame (ORF). | | | |
|---|---|---|---|
| EDG | Chromosomal localisation spliced/unspliced in ORF | | according BAC accession number: |
| EDG1 | 1p21.1-21.3 | unspliced | AL161741 |
| EDG2 | 9q31.1-32/ /18p11.3 | spliced | AL157881/ /AP000882 |
| EDG3 | 9q22.1-q22.2 | unspliced | |
| EDG4 | 19p12 | spliced | NT_000939 |
| EDG5 | 19 | unspliced | AC011511 |
| EDG6 | 19p13.3 | unspliced | AC011547 |
| EDG7 | 1 p22.3-31.2 | spliced | AL139822 |
| EDG8 | 19 | unspliced | AC011461 |

### Examples

### Example 1: Molecular cloning of the human EDG8 receptor.

As the putative human EDG8 sequence is intronless, we cloned the receptor from human genomic DNA (CLONTECH, Palo Alto, CA, 94303-4230) via polymerase chain reaction (PCR). PCR conditions, established to amplify the EDG8 sequence were 94°C, 1 min followed by 35 cycles of 94°C, 30sec, 68C, 3 min, using GC-Melt Kit (CLONTECH, Palo Alto, CA). Primers designed to amplify the EDG8 sequence contained a HindIII site in the forward, and a EcoRl site in the reverse primer, respectively. The 1197 bp PCR product was cloned into the pCDNA3.1(+) mammalian expression vector (Invitrogen, Carlsbad, California) and sequenced in both directions.

### Example 2: Cell culture and Transfection.

CHO-K1 cells were grown in basal ISCOVE medium supplemented with 10% fetal bovine serum at 37°C in a humidified 5% CO2 incubator. For transfections, 2 x 10⁵ cells were seeded into 35-mm dishes. About 24 hr later cells were transiently transfected at 50-80% confluency with the indicated receptor and G-protein constructs (1µg of plasmid DNA each) using the Lipofectamine transfection reagent and the supplied protocol (GIBCO). 18-24 hr after transfection cells were seeded into 96well plates at a density of 50.000 cells per well and cultured for 18-24 additional hr until used in the functional FLIPR assays.
The cDNA for Gα16 was cloned from TF1 cells by RT-PCR and ligated into the pCDNA1.1 mammalian expression vector (Invitrogen). Murine wild type Gαq was cloned from cells by RT-PCR and inserted into the BamHI-Nsil-sites of pCDNA1.1. To create the C-terminally modified Gα_{qi5} subunit, in which the last five aa of wt Gαq were replaced with the correspoding Gαᵢ sequence, a 175-bp Bglll-Nsil fragment was replaced, in a two piece ligation, with a synthetic DNA fragment, containing the desired codon changes. The correctness of all PCR-derived sequences was verified by sequencing in both directions.

### Example 3: Fluorometric Imaging Plate Reader (FLIPR) Assay.

Twenty-four hours after transfection, cells were splitted into 96-well, black-wall microplates (Corning) at a density of 50,000 cells per well. 18-24 hr later, cells were loaded with 95µl of HBSS containing 20 mM Hepes, 2.5 mM probenecid, 4 µM fluorescent calcium indicator dye Fluo4 (Molecular Probes) and 1% fetal bovine serum for 1 h(37°C, 5% CO₂). Cells were washed three times with HBSS containing 20 mM Hepes and 2.5 mM probenecid in a cell washer. After the final wash, the solution was aspirated to a residual volume of 100 µl per 96 well. Lipid ligands were dissolved in DMSO as 2 mM stock solutions (treated with ultrasound when necessary) and diluted at least 1:100 into HBSS containing 20 mM HEPES, 2.5 mM probenecid and 0.4 mg/ml fatty acid free bovine serum albumine. Lipids were aliquoted as 2X solutions into a 96 well plate prior to the assay. The fluorometric imaging plate reader (FLIPR, Molecular Devices) was programmed to transfer 100 µl from each well of the ligand microplate to each well of the cellplate and to record fluorescence during 3 min in 1 second intervals during the first minute and 3 second intervals during the last two minutes. Total fluorescence counts from the 18-s to 37-s time points are used to determine agonist activity. The instrument software normalizes the fluorescent reading to give equivalent initial readings at time zero.

### Example 4: Northern Blot analysis.

Human multiple tissue Northern blots were purchased from CLONTECH (Palo Alto, CA, 94303-4230, USA) antisense RNA probes were generated by subcloning nucleotides 279-1197 of the coding region into the Bam HI-Eco RI sites of the expression vector PSPT18 (Roche Diagnostics, Mannheim, Germany) and subsequent random priming with a DIG-RNA Labeling kit (Roche Diagnostics, Mannheim, Germany), using T7 RNA polymerase. Hybridization was carried out at 68°C for 16 h in hybridization buffer (Dig Easy Hyb Roche Diagnostics, Mannheim, Germany). Each blot was washed , blocked and detected as indicated in the standard protocol with the DIG Wash and Block Buffer set (Roche Diagnostics, Mannheim, Germany) and treated with 1 ml CSPD ready-to-use(Roche Diagnostics, Mannheim, Germany) for 15 min , 37°C and developed for 5 min on the Lumiimager (Roche). Finally, each blot was stripped (50 % formamid,5% SDS, 50 mM Tris/HCl pH 7,5 ; 80° C, 2x 1 hour) and rehybridized with a GAPDH antisense RNA probe as an internal standard.

### Example 5: RNA Extraction and RT-PCR.

RNA was prepared from different endothelial cell lines (HUVECS, HCAEC, HMVEC-L, HPAEC) using the TRlzol reagent (Hersteller, Lok.). Briefly, for each endothelial cell line, cells of a subconfluent 25 cm2 tissue culture flask were collected in 2,5ml TRlzol and total RNAs were extracted according to the supplied protocol. The purity of the RNA preparation was checked by veryfying the absence of genomic DNA. An aliquot of RNA, corresponding to ~5µg, was used for the cDNA generation using MMLV reverse transcriptase and the RT-PCR kit from STRATAGENE. RT-PCR was carried out in a volume of 50 µl, the RT-PCR conditions were set to 65°C for 5 min, 15min at RT, 1 hour at 37°C, 5 min at 90°C, chill on ice.
The cDNA templates for the PCR reactions (35 cycles of 94°C for 30 sec, 68°C for 3 min) were the reverse transcribed products of RNAs isolated from human endothelial cell lines (HUVECS,HCAEC, HMVEC-L, HPAEC). Typically, 1-5 µl of reverse transcribed cDNAs were used as templates for the PCR reactions.

### Example 6: Sources of materials.

1-oleoyl-LPA, sphingosin 1-phosphate (S1P), dihydrosphingosin 1-phosphate (DHS1 P), lysophosphatidylcholine (LPC), sphingosylphosphorylcholine (SPC) and fatty acid free BSA were from SIGMA (P.O.Box 14508, St. Louis, Missouri 63178). CHO-K1 cells were obtained from the American Type culture collection (ATCC, Manassas, Virginia), cell culture media and sera from GIBCO BRL (Gaithersburg, MD), the Ca fluorescent dye FLUO4 and pluronic acid from Molecular devices (Sunnyvale CA 94089-1136,USA) human northern blot membrane from CLONTECH (1020 East Meadow Circle, Palo Alto, California 94303-4230, USA.), commercially available cDNAs (heart, fetal heart, left atrium, left ventricle, kidney, brain, liver, lung, aorta) from Invitrogen, oligonucleotides from MWG-Biotech AG (Ebersberg, Germany), the RT-PCR kit from SIGMA, the GC-melt PCR kit from Clontech (Palo Alto, CA), the expression plasmid pcDNA3.1 for EDG8 and pCDNA1.1 for expression of G-protein α subunits from Invitrogen (Carlsbad, CA 92008), competent DH5α from GIBCO and MC 1063 from Invitrogen.

### References

An S, BleuT, Hallmark OG, and Goetzl EJ (1998) Characterization of a novel subtype of human G protein coupled receptor for lysophosphatidic acid. J Bio. Chem 273:7906-7910
An S, Bleu T, and Zheng Y (1999) Transduction of intracellular calcium signals through G protein-mediated activation of phospholipase C by recombinant sphingosine 1-phosphate receptors. Mol Pharmaco. 55:787-794
An S, Zheng Y, and Bleu T (2000) Sphingosine 1-phosphate induced cell proliferation, survival, and related signaling events mediated ba G protein coupled receptors edg3 and edg5. J Biol Chem 275:288-296
Ancellin N and Hla T (1999) Differential pharmacological properties and signal transduction of the sphingosine 1-phosphate receptors EDG-1, EDG-3, and EDG-5. JJ Biol Chem 274:18997-19002
Bandoh K, Aoki J, Hosono H, Kobayashi S, Kobayashi T, Murakami-Murofushi K, Tsujimoto M, Arai H, and Inoue K (2000) J Biol Chem 274:27776-27785
Bünemann M, Liliom K, Brandts BK, Pott L, Tseng JL, Desiderio DM, Sun G, Miller D, and Tigyi G (1996) A novel membrane receptor with high affinity for lysosphingomyelin and sphingosine 1-phosphate in atrial myocytes. EMBO J 15:5527-5534
Chao CP, Laulederkind SJ, and Ballou LR (1994) Sphingosine mediated phosphatidyl metabolism and calcium mobilization. J Biol Chem 269:5849-5856
Durieux ME, Carlisle SJ, Salafranca MN, and Lynch KR (1993) Responses to sphingosine 1-phosphate in X. laevis oocytes: similarities with lysophosphatidic acid signalling. Am J Physiol 264:C1360-C1364
   Glickman M, Malek RL, Kwitek-Black AE, Jacob HJ, and Lee NH (1999) Molecular cloning, tissue-specific ecpression, and chromosomal localization of a novel nerve growth factor-regulated G-protein-coupled receptor, nrg-1. Molecular and Cellular Neuroscience 14:141-152
Gohla A, Harhammer R, and Schultz G (1998) The G protein G13 but not G12 mediates signalling from lysophosphatidic acid receptor via epidermal growth factor to Rho.. J Biol Chem 273:653-4659
Gohla A, Offermanns S, Wilkie TM, and Schultz G (1999) Differential involvement of G 12 and G 13 in receptor-mediated stress fiber formation. J Biol Chem 274:17901-17907
Gonda K, Okamoto H, Takuwa N, Yatomi Y, Okazaki H, Sakrai T, Kimura S, Sillard R, Harii K, and Takuwa Y (1999) The novel sphingosine 1-phosphate receptor AGR16 is coupled via pertussis toxin-sensitive and -insensitive G-proteins to multiple signalling pathways. Biochem J 337:67-75
Gosh TK, Bian J, and Gill DL (1994) Sphingosine 1-phosphate generated in the endoplasmic reticulum membrane activates release of stored Calcium. J Biol Chem 269:22628-22635
Gueguen G, Gaige B, Grevy JM, Rogalle P, Bellan J, Wilson M, Klaebe A, Pont F, Simon MF, and Chap H (1999) Structure-activity analysis of the effects of lysophosphatidic acid on platelet aggregation. Biochemistry 38:8440-8450
Hisano N, Yatomi Y, Satoh K, Akimoto S, Mitsumata M, Fujino MA, and Ozaki Y (1999) Induction and suppression of endothelial cell apoptosis by sphingolipids: a possible in vitro model for cell-cell interactions between platelets and endothelial cells. Blood 93:4293-4299
Hla T and Maciag T (1990) An abundant transcript induced in differentiating human endothelial cells encodes a polypeptide with structural similarities to G-protein-coupled receptors. J. Biol. Chem. 265: 9308-9313
Hla T, Lee M, Ancellin N, Liu CH, Thangada S, Thompson BD, and Kluk M (1999) Sphingosine-1-phosphate: extracellular mediator or intracellular second messenger? Biochem Pharm 58:201-207
lm DS, Heise CE, Harding MA, George SR, O'Dowd BF, Theodorescu D, and Lynch KR (2000) Molecular cloning and characterization of a lysophosphatidic acid receptor, edg7, expressed in prostate. Mol Pharmacol 57:753-759
Jalink K, Moolenaar WH, and van Dujin B (1993) Lysophosphatidic acid is a chemoattractant for dictyostelium discoideum amoebae. Proc Natl Acad Sci USA 90:1857-1861
Jalink K, Hordijk PL, and Moolenaar WH (1994) Growth factor-like effects of lysophosphatidic acid, a novel lipid mediator. Biochim Biophys Acta 1198:185-196
Kon J, Sato K, Watanabe T, Tomura H, Kuwabara A, Kimura T, Tamama K, Ishizuka T, Murata N, Kanda T, Kobayashi I, Ohta H, Ui M, and Okajima F (1999) Comparison of intrinsic activities of the putative sphingosine 1-phosphate receptor subtypes to regulate several signalling pathways in their cDNA-transfected chinese hamster ovary cells. J Biol Chem 274:23940-23947
Kostenis E, Degtyarev MY, Conklin BR, and Wess J (1997) The N-terminal extension of G q is critical for constraining the selectivity of receptor coupling. J Biol Chem 272:19107-19110
Lee MJ, Evans M, and Hla T (1996) The inducible G protein-coupled receptor edg-1 signals via the G(i)/mitogen-activated protein kinase pathway. J Biol Chem 271:11272-11279
Lee MJ, Van Brocklyn JR, Thangada S, Liu CH, Hand AR, Menzeleev R, Spiegel S, and Hla T (1998a) Sphingosine 1-phosphate as a ligand for the G protein coupled receptor EDG-1. Science 279:1552-1555
Lee MJ, Thangada S, Liu CH, Thompson BD, and Hla T (1998) Lysophosphatidic acid stimulates the G-protein-coupled receptor edg-1 as a low affinity agonist. J Biol Chem 273:22105-22112
Lee MJ, Thangada S, Claffey KP, Ancellin N, Liu CH, Kluk M, Volpi M, Sha'afi RI, and Hla T (1999) Vascular endothelial cell adherens junction assembly and morphogenesis induced by sphingosine 1-phosphate. Cell 99:301-312
Lynch K and lm DS Life on the edg. Trends Pharmacol Sci 20:473-475
Mattie M, Brooker G, and Spegel S (1994) Sphingosine 1-phosphate, a putative second messenger, mobilizes Calcium from internal stores via an inositoltriphosphate-independent pathway. J Biol Chem 269:3181-3188
Meyer zu Heringdorf D, van Koppen CJ, Windorfer B, Himmel HM, and Jakobs KH (1997) Calcium signalling by G protein coupled sphingolipid receptors in bovine aortic endothelial cells. Naunyn-Schmiedeberg's Arch Pharmacol 354:397-403
Moolenaar WH, Kranenburg O, Postma FR, and Zondag GCM (1997) Lysophosphatidic acid: G-protein signalling and cellular responses. Current opinion in cell biology 9:168-173
Morris AJ (1999) One wheel on my wagon: lysolipid phosphate signalling. Trends Pharmacol Sci 20:393-395
Noh SJ, Kim MJ, Shim S, and Han JK (1998) Different signalling pathway between sphingosine 1-phosphate and lysophosphatidic acid in Xenopus oocytes: Functional coupling of the sphingosine 1-phosphate receptor to PLCx-beta in Xenopus oocytes. J Cell Physiol 176:412-423
Okajima F, Tomura H, Sho K, Nochi H, Tamoto K, and Kondo Y (1996) Involvement of pertussis toxin-sensitive GTP-binding proteins in sphingosine 1-phosphate induced activation of phospholipase C-Ca2+ system in HL60 leukemia cells. FEBS Lett 379:260-264
Okamoto H, Takuwa N, Gonda K, Okazaki H, Chang K, Yatomi Y, Shigematsu H, and Takuwa Y (1998) EDG1 is a functional sphingosine 1-phosphate receptor that is linked via a Gi/o to multiple signalling pathways, including phospholipase C activation, Ca2+-mobilization, ras-mitogen-activated protein kinase activation, and adenylate cyclase inhibition. J Biol Chem 273:27104-27110
Postma R, Jalink K, Hengeveld T, and Moolenaar WH (1996) Sphingosine 1-phosphate rapidly induces Rho-dependent neurite retraction: Action through a specific cell surface receptor. EMBO J 15:2388-2392
Rizza C, Leitinger N, Yue J, Fischr DJ, Wang D, Shih PT, Lee H, tigyi G, and Berliner JA (1999) Laboratory Investigation 79:1227-1235
Sadahira Y, Ruan F, Hakomori S, and Igarashi Y (1992) Sphingosine 1-phosphate, a specific endogeneous signalling molecule controlling cell motility and tumor cell invasiveness. Proc Natl Acad Sci USA 89:9686-9690
   Schulze C, Smales C, Rubin LL, and Staddon JM (1997) Lysophosphatidic acid increases tight junction permeability in cultured brain endothelial cells. J Neurochem 68:991-1000
Siess W, Zangl KJ, Essler M, Bauer M, Brandl R, CorrinthC, Bittman R, Tigyi G, and Aepfelbacher M (1999) Lysophosphatidic acid mediates the rapid activation of platelets and endothelial cells by mildly oxidized low density lipoprotein and accumulates in human atherosclerotic lesions. Proc Natl Acad Sci USA 96:6931-6936
Tokumura A, Fukuzawa K, Yamada S, and Tsukatani H (1980) Stimulatory effect of lysophosphatidic acids on uterine smooth muscles of non-pregnant rats. Arch Int Pharmacodyn Ther 245:74-83
   Tokumura A,Yotsumoto T, Masuda Y, and Tanaka S (1995) Vasopressor effect of lysophosphatidic acid on spontaneously hypertensive rats and wistar kyoto rats. Research Communications in Molecular Patology and Pharmacology 90:96-102
Törnquist K, Saarinen P, Vainio M, and Ahlstrom M (1997) Sphingosine 1-phosphate mobilizes sequestered Calcium, activates calcium entry, and stimulates desoxyribonucleic acid synthesis in thyroid FRTL-5 cells. Endocrinology 138:4049-4057
Van Brocklyn JR, Graler MH, Bernhardt G, Hobson JP, Lipp M, Spiegel S (2000) Sphingosine-1-phosphate is a ligand for the G protein-coupled receptor EDG-6. Blood 95(8):2624-2629
Van Koppen C, Meyer zu Heringdorf D, Laser KT, Zhang C, Jakobs KH, Bunemann M, and Pott L (1996) Activation of a high affinity Gi protein-coupled plasma membrane receptor by sphingosine 1-phosphate. J Biol Chem 271:2082-2087
Wu J, Spiegel S, and Sturgill TW (1995) Sphingosine 1-phosphate rapidly activates the mitogen activated protein kinase pathway by a G protein-dependent mechanism. J Biol Chem 270:11484-11488
Xia P, Wang L, Gamble JR, and Vadas MA (1999) Activation of sphingosine kinase by tumor necrosis factor- inhibits apoptosis in human endothelial cells. J Biol Chem 274:34499-34505
Yamazaki Y, Kon J, Sato K, Tomura H, Sato M, Yoneya T, Okazaki H, Okajima F, Ohta H(2000) Edg-6 as a putative sphingosine 1-phosphate receptor coupling to Ca(2+) signaling. Biochem Biophys Res Commun 268(2):583-589
Yatomi Y, Yamamura S, Ruan F, and Igarashi Y (1997a) Sphingosine 1-phosphate induces platelet activation through an extracellular action and shares a platelet surface receptor with lysophosphatidic acid. J Biol Chem 272:5291-5297
Yatomi Y, Igarashi Y, Yang L, Hisano N,Qi R, Asazuma N, Satoh K, Ozaki Y, and Kume S (1997b) J Biochem (Tokyo) 12:969-973
Zhang H, Desai NN, Olivera A, Seki T, Brooker G, and Spiegel S (1991) Sphingosine 1-phosphate, a novel lipid, involved in cellular proliferation. J Cell Biol 114:155-167

### List of non-standard abbreviations:

S1P, sphingosine 1-phosphate; LPA, lysophosphatidic acid; dHS1P, dihydro sphingosine 1-phosphate; SPC, sphingosylphosphorylcholine; LPC, lysophosphatidylcholine; GPCR, G-protein-coupled receptor; G-protein, guanine nucleotide-binding protein; [Ca²⁺]ᵢ, intracellular Calcium concentration, RT-PCR, reverse transcription polymerase chain reaction; bp, base pair; ORF, open reading frame; EST, expressed sequence tag; FAF-BSA, fatty acid free bovine serum albumine; HUVECS. Human umbilical vein endothelial cells; HCAEC, human coronary artery endothelial cells; HMVEC-L, human microvascular endothelial cells from lung; HPAEC, human pulmonary artery endothelial cells.

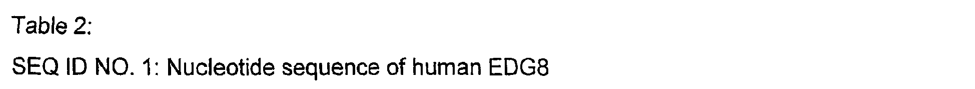

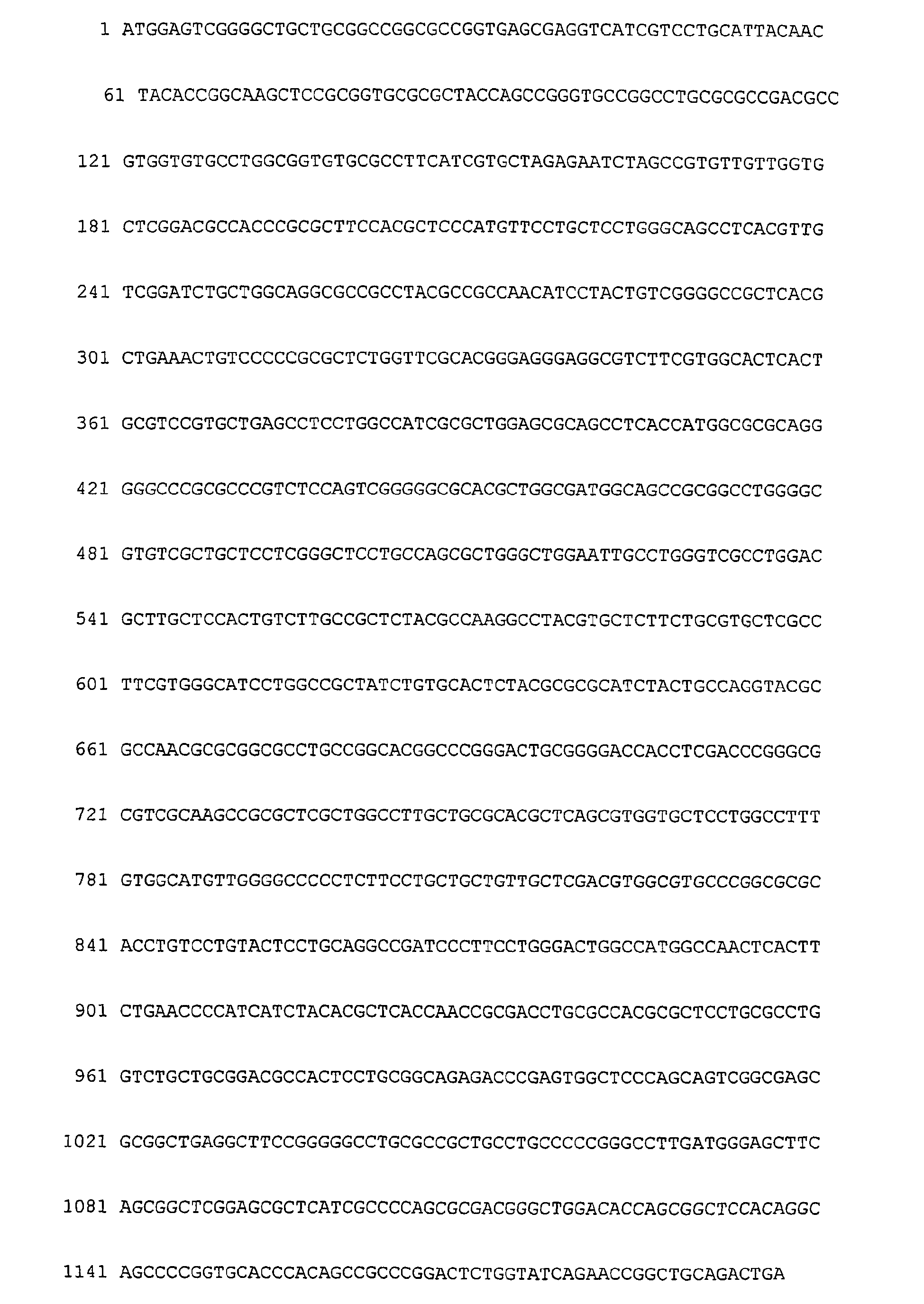

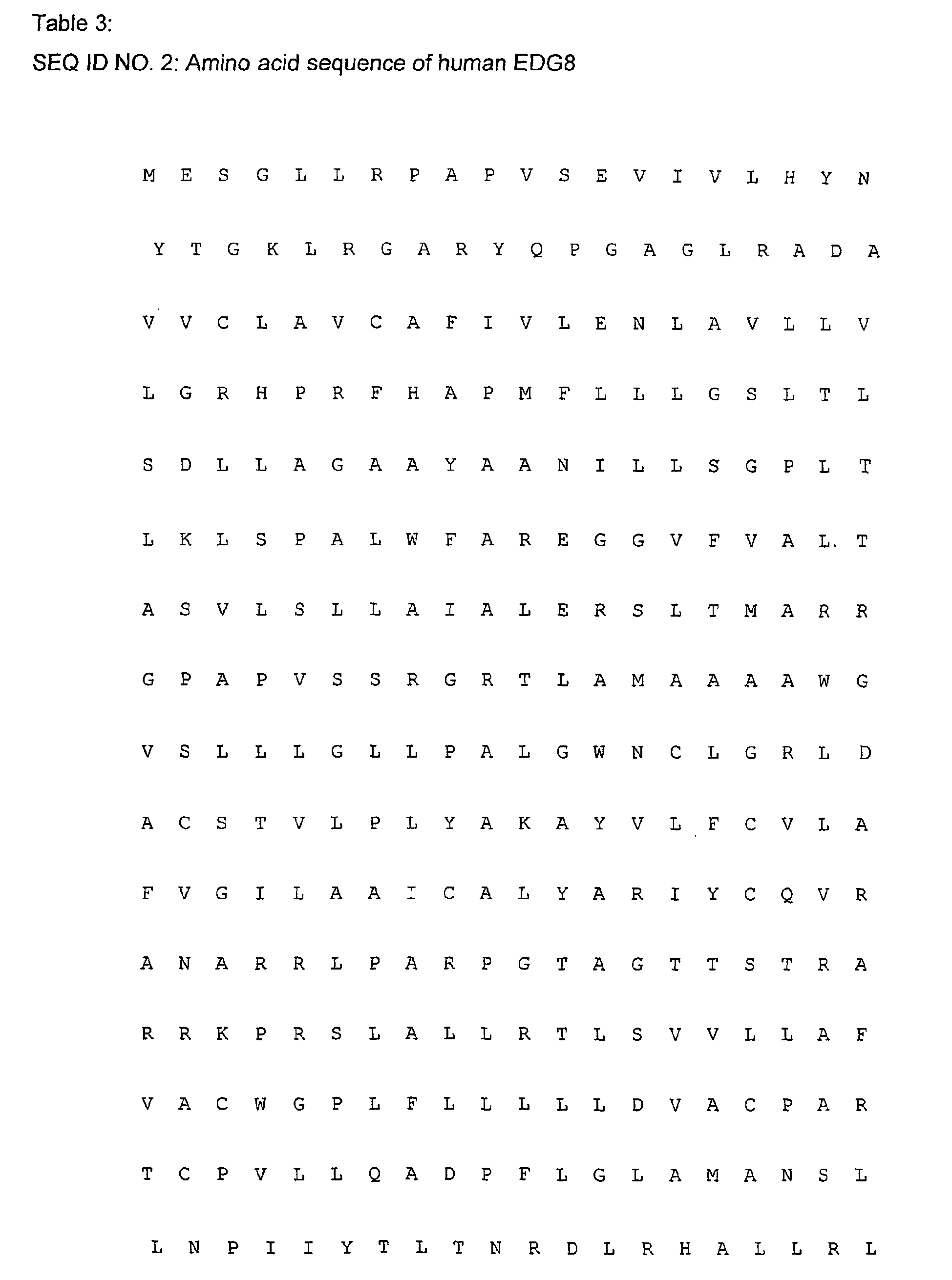

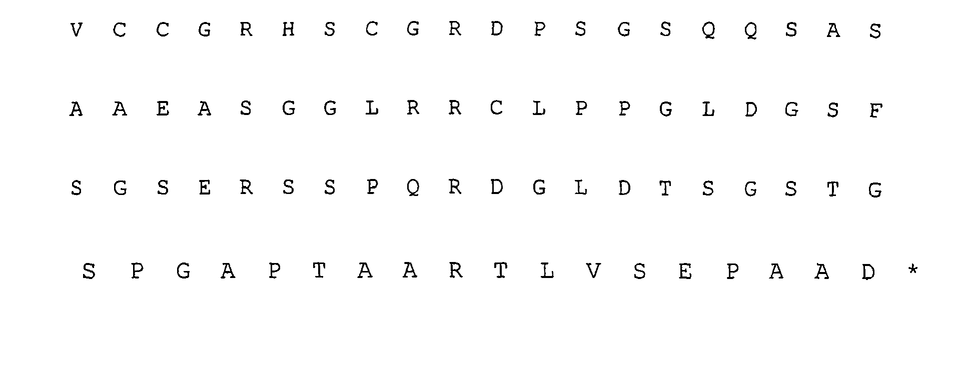

## Claims

1. A method for identifying a compound which binds to EDG8 polypeptide comprising an amino acid sequence according to SEQ ID NO: 2 or encoded by a polynucleotide sequence according to SEQ ID NO: 1 in an antagonistic manner with respect to sphingosine 1-phosphate (S1P), lysophosphatidic acid (LPA) and/or dihydrosphingosine 1-phosphate (DHS1P) comprising
a] contacting a cell expressing said EDG8 polypeptide and cotransfected with Gα₁₆ or Gα_{qi5}, or a part of such a cell, with S1P, and/or LPA and/or DHS1P and a candidate compound, and
b] assessing the ability of said compound to bind to said EDG8 polypeptide in an antagonistic manner with respect to S1P, LPA and/or DHS1P.

2. The method as claimed in claim 1 which further includes determining whether the candidate compound effects a signal generated by activation of said EDG8 polypeptide in an antagonistic manner at the surface of the cell, wherein a candidate which effects production of said signal is identified as an antagonist.

3. The method as claimed in claim 1 wherein the polynucleotide sequence is part of an expression vector.

## Patentansprüche

1. Verfahren zur Identifizierung einer Verbindung, die an das eine Aminosäuresequenz gemäß SEQ ID NO: 2 umfassende bzw. von einer Polynukleotidsequenz gemäß SEQ ID NO: 1 codierte Polypeptid EDG8 auf antagonistische Weise in bezug auf Sphingosin-1-phosphat (S1P), Lysophosphatidinsäure (LPA) und/oder Dihydrosphingosin-1-phosphat (DHS1P) bindet, wobei man in dem Verfahren
a] eine das EDG8-Polypeptid exprimierende und mit Gα₁₆ oder Gα_{qi5} cotransfizierte Zelle oder einen Teil einer derartigen Zelle mit S1P und/oder LPA und/oder DHS1P sowie einer Kandidatenverbindung in Kontakt bringt und
b] die Fähigkeit der Verbindung, an das EDG8-Polypeptid auf antagonistische Weise in bezug auf S1P, LPA und/oder DHS1P zu binden, beurteilt.

2. Verfahren nach Anspruch 1, bei dem man ferner bestimmt, ob die Kandidatenverbindung ein durch Aktivierung des EDG8-Polypeptids erzeugtes Signal auf antagonistische Weise an der Oberfläche der Zelle herbeiführt, wobei ein die Bildung des Signals herbeiführender Kandidat als Antagonist identifiziert wird.

3. Verfahren nach Anspruch 1, wobei die Polynukleotidsequenz Teil eines Expressionsvektors ist.

## Revendications

1. Procédé d'identification d'un composé qui se lie au polypeptide EDG8, comprenant une séquence d'acides aminés selon SEQ ID NO : 2 ou codé par une séquence polynucléotidique selon SEQ ID NO : 1, de manière antagoniste par rapport à la sphingosine 1-phosphate (S1P), à l'acide lysophosphatidique (LPA) et/ou à la dihydrosphingosine 1-phosphate (DHS1P), comprenant les étapes consistant à
a) mettre en contact une cellule exprimant ledit polypeptide EDG8 et co-transfectée avec Gα₁₆ ou Gα_{qi5}, ou une partie d'une telle cellule, avec S1P et/ou LPA et/ou DHS1P et un composé candidat, et
b) évaluer l'aptitude dudit composé à se lier audit polypeptide EDG8 de manière antagoniste par rapport à S1P, LPA et/ou DHS1P.

2. Procédé selon la revendication 1, qui inclut en outre l'étape consistant à déterminer si le composé candidat conduit à un signal généré par l'activation dudit polypeptide EDG8 de manière antagoniste à la surface de la cellule, **caractérisé en ce qu'**un candidat qui conduit à la production dudit signal est identifié en tant qu'antagoniste.

3. Procédé selon la revendication 1, **caractérisé en ce que** la séquence polynucléotidique fait partie d'un vecteur d'expression.


### SEQUENCE LISTING

<110> Aventis Pharma Deutschland GmbH
<120> EDG8 receptor, its preparation and use
<130> AVE D-2000/A024
<140>
   <141>
<150> 00108858.2
   <151> 2000-04-26
<150> 00116589.3
   <151> 2000-08-01
<160> 2
<170> Patent In Ver. 2.1
<210> 1
   <211> 1197
   <212> DNA
   <213> Homo sapiens
<400> 1
   atggagtcgg ggctgctgcg gccggcgccg gtgagcgagg tcatcgtcct gcattacaac 60
   tacaccggca agctccgcgg tgcgcgctac cagccgggtg ccggcctgcg cgccgacgcc 120
   gtggtgtgcc tggcggtgtg cgccttcatc gtgctagaga atctagccgt gttgttggtg 180
   ctcggacgcc acccgcgctt ccacgctccc atgttcctgc tcctgggcag cctcacgttg 240
   tcggatctgc tggcaggcgc cgcctacgcc gccaacatcc tactgtcggg gccgctcacg 300
   ctgaaactgt cccccgcgct ctggttcgca cgggagggag gcgtcttcgt ggcactcact 360
   gcgtccgtgc tgagcctcct ggccatcgcg ctggagcgca gcctcaccat ggcgcgcagg 420
   gggcccgcgc ccgtctccag tcgggggcgc acgctggcga tggcagccgc ggcctggggc 480
   gtgtcgctgc tcctcgggct cctgccagcg ctgggctgga attgcctggg tcgcctggac 540
   gcttgctcca ctgtcttgcc gctctacgcc aaggcctacg tgctcttctg cgtgctcgcc 600
   ttcgtgggca tcctggccgc tatctgtgca ctctacgcgc gcatctactg ccaggtacgc 660
   gccaacgcgc ggcgcctgcc ggcacggccc gggactgcgg ggaccacctc gacccgggcg 720
   cgtcgcaagc cgcgctcgct ggccttgctg cgcacgctca gcgtggtgct cctggccttt 780
   gtggcatgtt ggggccccct cttcctgctg ctgttgctcg acgtggcgtg cccggcgcgc 840
   acctgtcctg tactcctgca ggccgatccc ttcctgggac tggccatggc caactcactt 900
   ctgaacccca tcatctacac gctcaccaac cgcgacctgc gccacgcgct cctgcgcctg 960
   gtctgctgcg gacgccactc ctgcggcaga gacccgagtg gctcccagca gtcggcgagc 1020
   gcggctgagg cttccggggg cctgcgccgc tgcctgcccc cgggccttga tgggagcttc 1080
   agcggctcgg agcgctcatc gccccagcgc gacgggctgg acaccagcgg ctccacaggc 1140
   agccccggtg cacccacagc cgcccggact ctggtatcag aaccggctgc agactga    1197
<210> 2
   <211> 398
   <212> PRT
   <213> Homo sapiens
<400> 2
   Met Glu Ser Gly Leu Leu Arg Pro Ala Pro Val Ser Glu Val Ile Val
    1               5                  10                  15
   Leu His Tyr Asn Tyr Thr Gly Lys Leu Arg Gly Ala Arg Tyr Gln Pro
               20                  25                  30
   Gly Ala Gly Leu Arg Ala Asp Ala Val Val Cys Leu Ala Val Cys Ala
           35                  40                  45
   Phe Ile Val Leu Glu Asn Leu Ala Val Leu Leu Val Leu Gly Arg His
       50                  55                  60
   Pro Arg Phe His Ala Pro Met Phe Leu Leu Leu Gly Ser Leu Thr Leu
   65                  70                  75                  80
   Ser Asp Leu Leu Ala Gly Ala Ala Tyr Ala Ala Asn Ile Leu Leu Ser
                   85                  90                  95
   Gly Pro Leu Thr Leu Lys Leu Ser Pro Ala Leu Trp Phe Ala Arg Glu
               100                 105                 110
   Gly Gly Val Phe Val Ala Leu Thr Ala Ser Val Leu Ser Leu Leu Ala
           115                 120                 125
   Ile Ala Leu Glu Arg Ser Leu Thr Met Ala Arg Arg Gly Pro Ala Pro
       130                 135                 140
   Val Ser Ser Arg Gly Arg Thr Leu Ala Met Ala Ala Ala Ala Trp Gly
   145                 150                 155                 160
   Val Ser Leu Leu Leu Gly Leu Leu Pro Ala Leu Gly Trp Asn Cys Leu
                   165                 170                 175
   Gly Arg Leu Asp Ala Cys Ser Thr Val Leu Pro Leu Tyr Ala Lys Ala
               180                 185                 190
   Tyr Val Leu Phe Cys Val Leu Ala Phe Val Gly Ile Leu Ala Ala Ile
           195                 200                 205
   Cys Ala Leu Tyr Ala Arg Ile Tyr Cys Gln Val Arg Ala Asn Ala Arg
       210                 215                 220
   Arg Leu Pro Ala Arg Pro Gly Thr Ala Gly Thr Thr Ser Thr Arg Ala
   225                 230                 235                 240
   Arg Arg Lys Pro Arg Ser Leu Ala Leu Leu Arg Thr Leu Ser Val Val
                   245                 250                 255
   Leu Leu Ala Phe Val Ala Cys Trp Gly Pro Leu Phe Leu Leu Leu Leu
               260                 265                 270
   Leu Asp Val Ala Cys Pro Ala Arg Thr Cys Pro Val Leu Leu Gln Ala
           275                 280                 285
   Asp Pro Phe Leu Gly Leu Ala Met Ala Asn Ser Leu Leu Asn Pro Ile
       290                 295                 300
   Ile Tyr Thr Leu Thr Asn Arg Asp Leu Arg His Ala Leu Leu Arg Leu
   305                 310                 315                 320
   Val Cys Cys Gly Arg His Ser Cys Gly Arg Asp Pro Ser Gly Ser Gln
                   325                 330                 335
   Gln Ser Ala Ser Ala Ala Glu Ala Ser Gly Gly Leu Arg Arg Cys Leu
               340                 345                 350
   Pro Pro Gly Leu Asp Gly Ser Phe Ser Gly Ser Glu Arg Ser Ser Pro
           355                 360                 365
   Gln Arg Asp Gly Leu Asp Thr Ser Gly Ser Thr Gly Ser Pro Gly Ala
       370                 375                 380
   Pro Thr Ala Ala Arg Thr Leu Val Ser Glu Pro Ala Ala Asp
   385                 390                 395